# EUROPEAN PATENT SPECIFICATION

(11) **EP 1 165 738 B1**
(45) Date of publication and mention of the grant of the patent: **27.07.2005**
(21) Application number: 00918830.1
(22) Date of filing: 22.03.2000
(51) Int. Cl.: C11D 3/395, C07D 213/38, C07D 213/55, C07D 401/14, C07F 15/02, C07F 13/00, D06L 3/02

(54) **COMPOSITION AND METHOD FOR BLEACHING A SUBSTRATE**
ZUSAMMENSETZUNG UND VERFAHREN ZUM BLEICHEN EINES SUBSTRATS
COMPOSITION ET PROCEDE POUR BLANCHIR UN SUBSTRAT

(30) Priority: 01.04.1999 GB 9907714; 01.04.1999 GB 9907713; 01.09.1999 WO PCT/GB99/02876; 01.09.1999 WO PCT/GB99/02878; 29.02.2000 GB 0004850
(43) Date of publication of application: 02.01.2002
(73) Proprietor: UNILEVER PLC, London EC4P 4BQ (GB); UNILEVER N.V., 3013 AL Rotterdam (NL)
(72) Inventor: CARINA, Riccardo, Filippo, F-91940 Les Ulis (FR); FERINGA, Bernard, Lucas, Stratingh Institute, 9747 AG Groningen (NL); HAGE, Ronald, Unilever Research Vlaardingen, 3133 AT Vlaardingen (NL); HEMMERT, Catherine, Lab.deChimie de Coord.du CNRS, 31077 Toulouse Cedex 4 (FR); KOEK, Jean, Hypolites, Unilever Res. Vlaardingen, 3133 AT Vlaardingen (NL); LACROIS, Rene, Marcel, Stratingh Institute, 9747 AG Groningen (NL); MEUNIER, Bernard, Lab. de Chimie de Coord. du CNRS, 31077 Toulouse Cedex 4 (FR); RENZ, Michael, Lab. de Chimie de Coord. du CNRS, 31077 Toulouse Cedex 4 (FR); ROELFES, Johannes, Gerhardus,, 8057 Zurich (CH); SCHUDDE, Ebe, Pieter, Stratingh Institute, 9747 AG Groningen (NL); THIJSSEN, Rob, Unilever Research Vlaardingen, 3133 AT Vlaardingen (NL); TWISKER, Robin, Stefan, Unilever Res. Vlaardingen, 3133 AT Vlaardingen (NL); ZONDERVAN, Charon, Agrotech.Research Institute ATO, 6700AA Wageningen (NL)
(74) Representative: Elliott, Peter William
(86) International application number: PCT/EP2000/002590
(87) International publication number: WO 2000/060044

(56) References cited:
- WO-A-00/12667
- WO-A-00/12808
- WO-A-95/27772
- WO-A-95/34628
- WO-A-96/06154
- WO-A-97/38074
- WO-A-97/48787
- WO-A-99/65905
- DE-A- 19 714 122

## Description

This invention relates to compositions and methods for catalytically bleaching substrates with atmospheric oxygen, more particularly using a defined class of ligand or complex as catalyst, and further relates to ligands and complexes useful in such compositions and methods. This invention also relates to a method of treating textiles, such as laundry fabrics, using the defined class of ligand or complex as catalyst, more specifically to a method whereby bleaching by atmospheric oxygen is catalysed after the treatment.

Peroxygen bleaches are well known for their ability to remove stains from substrates. Traditionally, the substrate is subjected to hydrogen peroxide, or to substances which can generate hydroperoxyl radicals, such as inorganic or organic peroxides. Generally, these systems must be activated. One method of activation is to employ wash temperatures of 60°C or higher. However, these high temperatures often lead to inefficient cleaning, and can also cause premature damage to the substrate.

A preferred approach to generating hydroperoxyl bleach radicals is the use of inorganic peroxides coupled with organic precursor compounds. These systems are employed for many commercial laundry powders. For example, various European systems are based on tetraacetyl ethylenediamine (TAED) as the organic precursor coupled with sodium perborate or sodium percarbonate, whereas in the United States laundry bleach products are typically based on sodium nonanoyloxybenzenesulfonate (SNOBS) as the organic precursor coupled with sodium perborate.

Precursor systems are generally effective but still exhibit several disadvantages. For example, organic precursors are moderately sophisticated molecules requiring multi-step manufacturing processes resulting in high capital costs. Also, precursor systems have large formulation space requirements so that a significant proportion of a laundry powder must be devoted to the bleach components, leaving less room for other active ingredients and complicating the development of concentrated powders. Moreover, precursor systems do not bleach very efficiently in countries where consumers have wash habits entailing low dosage, short wash times, cold temperatures and low wash liquor to substrate ratios.

Alternatively, or additionally, hydrogen peroxide and peroxy systems can be activated by bleach catalysts, such as by complexes of iron and the ligand N4Py (*i.e*. N. N-bis(pyridin-2-yl-methyl)-bis(pyridin-2-yl)methylamine) disclosed in WO95/34628, or the ligand Tpen (*i.e.* N, N, N', N'-tetra(pyridin-2-yl-methyl)ethylenediamine) disclosed in WO97/48787. According to these publications, molecular oxygen may be used as the oxidant as an alternative to peroxide generating systems. However, no role in catalysing bleaching by atmospheric oxygen in an aqueous medium is reported.

It has long been thought desirable to be able to use atmospheric oxygen (air) as the source for a bleaching species, as this would avoid the need for costly hydroperoxyl generating systems. Unfortunately, air as such is kinetically inert towards bleaching substrates and exhibits no bleaching ability. Recently some progress has been made in this area. For example, WO 97/38074 reports the use of air for oxidising stains on fabrics by bubbling air through an aqueous solution containing an aldehyde and a radical initiator. A broad range of aliphatic, aromatic and heterocyclic aldehydes is reported to be useful, particularly para-substituted aldehydes such as 4-methyl-, 4-ethyl- and 4-isopropyl benzaldehyde, whereas the range of initiators disclosed includes N-hydroxysuccinimide, various peroxides and transition metal coordination complexes.

However, although this system employs molecular oxygen from the air, the aldehyde component and radical initiators such as peroxides are consumed during the bleaching process. These components must therefore be included in the composition in relatively high amounts so as not to become depleted before completion of the bleaching process in the wash cycle. Moreover, the spent components represent a waste of resources as they can no longer participate in the bleaching process.

Accordingly, it would be desirable to be able to provide a bleaching system based on atmospheric oxygen or air that does not rely primarily on hydrogen peroxide or a hydroperoxyl generating system, and that does not require the presence of organic components such as aldehydes that are consumed in the process. Moreover, it would be desirable to provide such a bleaching system that is effective in aqueous medium.

It may also be noted that the known art teaches a bleaching effect only as long as the substrate is being subjected to the bleaching treatment. Thus, there is no expectation that hydrogen peroxide or peroxy bleach systems could continue to provide a bleaching effect on a treated substrate, such as a laundry fabric after washing and drying, since the bleaching species themselves or any activators necessary for the bleaching systems would be assumed to be removed from the substrate, or consumed or deactivated, on completing the wash cycle and drying.

It would be therefore also be desirable to be able to treat a textile such that, after the treatment is completed, a bleaching effect is observed on the textile. Furthermore, it would be desirable to be able to provide a bleach treatment for textiles such as laundry fabrics whereby residual bleaching occurs when the treated fabric has been treated and is dry.

We have found that a selected class of ligand or complex is surprisingly effective in catalysing the bleaching of substrates using atmospheric oxygen or air. Furthermore, we have found certain novel ligands which are useful in the bleaching of substrates using atmospheric oxygen or air.

Accordingly, in a first aspect, the present invention provides a bleaching composition comprising, in an aqueous medium, atmospheric oxygen and a ligand which forms a complex with a transition metal, the complex catalysing bleaching of a substrate by the atmospheric oxygen, wherein the aqueous medium is substantially devoid of peroxygen bleach or a peroxy-based or -generating bleach system. The medium is therefore preferably insensitive or stable to catalase, which acts on peroxy species.

In a second aspect, the present invention provides a method of bleaching a substrate comprising applying to the substrate, in an aqueous medium, a ligand which forms a complex with a transition metal, the complex catalysing bleaching of the substrate by atmospheric oxygen.

Furthermore, in a third aspect, the present invention provides the use of a ligand which forms a complex with a transition metal as a catalytic bleaching agent for a substrate in an aqueous medium substantially devoid of peroxygen bleach or a peroxy-based or - generating bleach system, the complex catalysing bleaching of the substrate by the atmospheric oxygen.

We have also found that certain ligands or complexes of this class are surprisingly effective in catalysing bleaching of the substrate by atmospheric oxygen after treatment of the substrate.

Accordingly, in a fourth aspect, the present invention provides a method of treating a textile by contacting the textile with a ligand which forms a complex with a transition metal, whereby the complex catalyses bleaching of the textile by atmospheric oxygen after the treatment.

In a fifth aspect, the present invention provides a dry textile having a ligand as defined above applied or deposited thereon, whereby bleaching by atmospheric oxygen is catalysed on the textile.

In further aspects, the present invention provides ligands and complexes, as defined further below.

Advantageously, the method according to the present invention permits all or the majority of the bleaching species in the medium (on an equivalent weight basis) to be derived from atmospheric oxygen. Thus, the medium can be made wholly or substantially devoid of peroxygen bleach or a peroxy-based or -generating bleach system. Furthermore, the complex is a catalyst for the bleaching process and, as such, is not consumed but can continue to participate in the bleaching process. The catalytically activated bleaching system of the type in accordance with the present invention, which is based on atmospheric oxygen, is therefore both cost-effective and environmentally friendly. Moreover, the bleaching system is operable under unfavourable wash conditions which include low temperatures, short contact times and low dosage requirements. Furthermore, the method is effective in an aqueous medium and is therefore particularly applicable to bleaching of laundry fabrics. Therefore, whilst the composition and method according to the present invention may be used for bleaching any suitable substrate, the preferred substrate is a laundry fabric. The bleaching method may be carried out by simply leaving the substrate in contact with the medium for a sufficient period of time. Preferably, however, the aqueous medium on or containing the substrate is agitated.

An advantage of the method according to the fourth aspect of the invention is that, by enabling a bleaching effect even after the textile has been treated, the benefits of bleaching can be prolonged on the textile. Furthermore, since a bleaching effect is conferred to the textile after the treatment, the treatment itself, such as a laundry wash cycle, may for example be shortened. Moreover, since a bleaching effect is achieved by atmospheric oxygen after treatment of the textile, hydrogen peroxide or peroxy-based bleach systems can be omitted from the treatment substance.

The ligand may be present as a preformed complex of a ligand and a transition metal. Alternatively, the composition may comprise a free ligand that complexes with a transition metal already present in the water or that complexes with a transition metal present in the substrate. The composition may also be formulated as a composition of a free ligand or a transition metal-substitutable metal-ligand complex, and a source of transition metal, whereby the complex is formed *in situ* in the medium.

The ligand forms a complex with one or more transition metals, in the latter case for example as a dinuclear complex. Suitable transition metals include for example: manganese in oxidation states II-V, iron II-V, copper I-III, cobalt I-III, titanium II-IV, tungsten IV-VI, vanadium II-V and molybdenum II-VI.

The ligand forms a complex of the general formula (A1):

[MₐLₖXₙ]Yₘ (A1)

in which:
M represents a metal selected from Mn(II)-(III)-(IV)-(V), Cu(I)-(II)-(III), Fe(II)-(III)-(IV)-(V), Co(I)-(II)-(III), Ti(II)-(III)-(IV), V(II)-(III)-(IV)-(V), Mo(II)-(III)-(IV)-(V)-(VI) and W(IV)-(V)-(VI), preferably selected from Fe(II)-(III)-(IV)-(V);
L represents a ligand as herein defined, or its protonated or deprotonated analogue;
X represents a coordinating species selected from any mono, bi or tri charged anions and any neutral molecules able to coordinate the metal in a mono, bi or tridentate manner, preferably selected from O²⁻, RBO₂²⁻, RCOO⁻, RCONR⁻, OH⁻, NO₃⁻, NO, S²⁻, RS⁻, PO₄³⁻, PO₃OR³⁻, H₂O, CO₃²⁻, HCO₃⁻, ROH, N(R)₃, ROO⁻, O₂²⁻, O₂⁻, RCN, Cl⁻, Br⁻, OCN⁻, SCN⁻, CN⁻, N₃⁻, F⁻, I⁻, RO⁻, ClO₄⁻, and CF₃SO₃⁻, and more preferably selected from O²⁻, RBO₂²⁻, RCOO⁻, OH⁻, NO₃⁻, S²⁻, RS⁻, PO₃⁴⁻, H₂O, CO₃²⁻, HCO₃⁻, ROH, N(R)₃, Cl⁻, Br⁻, OCN⁻, SCN⁻, RCN, N₃⁻, F⁻, I⁻, RO⁻, ClO₄⁻, and CF₃SO₃⁻;
Y represents any non-coordinated counter ion, preferably selected from ClO₄⁻, BR₄⁻, [MX₄]⁻, [MX₄]²⁻, PF₆⁻, RCOO⁻, NO₃⁻, RO⁻, N⁺(R)₄, ROO⁻, O₂²⁻, O₂⁻, Cl⁻, Br⁻, F⁻, I⁻, CF₃SO₃⁻, S₂O₆²⁻, OCN⁻, SCN⁻, H₂O, RBO₂²⁻, BF₄⁻ and BPh₄⁻, and more preferably selected from ClO₄⁻, BR₄⁻, [FeCl₄]⁻, PF₆⁻, RCOO⁻, NO₃⁻, RO⁻, N⁺(R)₄, Cl⁻, Br⁻, F, I⁻, CF₃SO₃⁻, S₂O₆²⁻, OCN⁻, SCN⁻, H₂O and BF₄⁻;
a represents an integer from 1 to 10, preferably from 1 to 4;
k represents an integer from 1 to 10;
n represents an integer from 1 to 10, preferably from 1 to 4;
m represents zero or an integer from 1 to 20, preferably from 1 to 8; and
each R independently represents a group selected from hydrogen, hyrdroxyl, -R' and -OR', wherein R'= alkyl, alkenyl, cycloalkyl, heterocycloalkyl, aryl, heteroaryl or a carbonyl derivative group, R' being optionally substituted by one or more functional groups E, wherein E independently represents a functional group selected from -F, -Cl, -Br, -I, -OH, -OR', -NH₂, -NHR', -N(R')₂, -N(R')₃⁺, -C(O)R', -OC(O)R', -COOH, -COO⁻ (Na⁺, K⁺), -COOR', -C(O)NH₂, -C(O)NHR', -C(O)N(R')₂, heteroaryl, -R', -SR', -SH, -P(R')₂, -P(O)(R')₂, -P(O)(OH)₂, -P(O)(OR')₂, -NO₂, -SO₃H, -SO₃⁻(Na⁺, K⁺), -S(O)₂R', -NHC(O)R', and -N(R')C(O)R', wherein R' represents cycloalkyl, aryl, arylalkyl, or alkyl optionally substituted by -F, -Cl, -Br, -I, -NH₃⁺, -SO₃H, -SO₃⁻(Na⁺, K⁺), -COOH, -COO⁻ (Na⁺, K⁺), -P(O)(OH)₂, or -P(O)(O⁻(Na⁺, K⁺))₂, and preferably each R independently represents hydrogen, optionally substituted alkyl or optionally substituted aryl, more preferably hydrogen or optionally substituted phenyl, naphthyl or C₁₋₄-alkyl.

The ligand L is of the general formula (I): 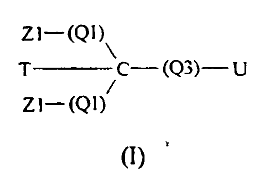 wherein
Z1 groups independently represent a coordinating group selected from hydroxy, amino, -NHR or -N(R)₂ (wherein R=C₁₋₆-alkyl), carboxylate, amido, -NH-C(NH)NH₂, hydroxyphenyl, a heterocyclic ring optionally substituted by one or more functional groups E or a heteroaromatic ring optionally substituted by one or more functional groups E, the heteroaromatic ring being selected from pyridine, pyrimidine, pyrazine, pyrazole, imidazole, benzimidazole, quinoline, quinoxaline, triazole, isoquinoline, carbazole, indole, isoindole, oxazole and thiazole;
Q1 and Q3 independently represent a group of the formula: 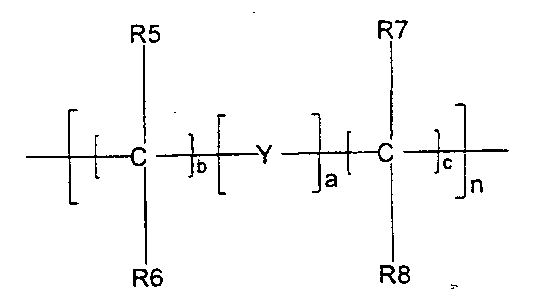 wherein
5 ≥ a+b+c ≥ 1; a=0-5; b=0-5; c=0.5; n=0 or 1 (preferably n=0);
Y independently represents a group selected from -O-, -S-, -SO-, -SO₂-, -C(O)-, arylene, alkylene, heteroarylene, heterocycloalkylene, -(G)P-, -P(O)- and -(G)N-, wherein G is selected from hydrogen, alkyl, aryl, arylalkyl, cycloalkyl, each except hydrogen being optionally substituted by one or more functional groups E;
R5, R6, R7, R8 independently represent a group selected from hydrogen, hydroxyl, halogen, -R and -OR, wherein R represents alkyl, alkenyl, cycloalkyl, heterocycloalkyl, aryl, heteroaryl or a carbonyl derivative group, R being optionally substituted by one or more functional groups E,
or R5 together with R6, or R7 together with R8, or both, represent oxygen,
or R5 together with R7 and/or independently R6 together with R8, or R5 together with R8 and/or independently R6 together with R7, represent C₁₋₆-alkylene optionally substituted by C₁₋₄-alkyl, -F, -Cl, -Br or -I;
T represents a non-coordinated group selected from hydrogen, hydroxyl, halogen, -R and -OR, wherein R represents alkyl, alkenyl, cycloalkyl, heterocycloalkyl, aryl, arylalkyl, heteroaryl or a carbonyl derivative group, R being optionally substituted by one or more functional groups E (preferably T= -H, -OH, methyl, methoxy or benzyl);
U represents either a non-coordinated group T independently defined as above or a coordinating group of the general formula (II), (III) or (IV): 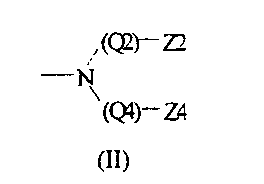 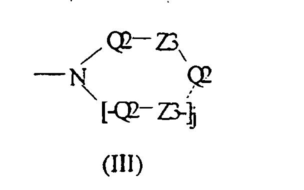 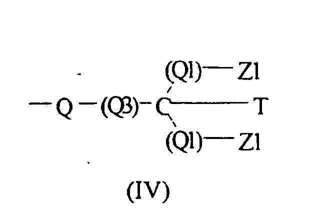 wherein
Q2 and Q4 are independently defined as for Q1 and Q3;
Q represents -N(T)- (wherein T is independently defined as above), or an optionally substituted heterocyclic ring or an optionally substituted heteroaromatic ring selected from pyridine, pyrimidine, pyrazine, pyrazole, imidazole, benzimidazole, quinoline, quinoxaline, triazole, isoquinoline, carbazole, indole, isoindole, oxazole and thiazole;
Z2 is independently defined as for Z1;
Z3 groups independently represent -N(T)- (wherein T is independently defined as above);
Z4 represents a coordinating or non-coordinating group selected from hydrogen, hydroxyl, halogen, -NH-C(NH)NH₂, -R and -OR, wherein R= alkyl, alkenyl, cycloalkyl, heterocycloalkyl, aryl, heteroaryl or a carbonyl derivative group, R being optionally substituted by one or more functional groups E, or Z4 represents a group of the general formula (IIa): 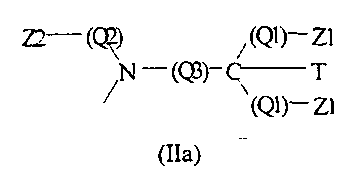 and
1≤j<4.

Preferably, Z1, Z2 and Z4 independently represent an optionally substituted heterocyclic ring or an optionally substituted heteroaromatic ring selected from pyridine, pyrimidine, pyrazine, pyrazole, imidazole, benzimidazole, quinoline, quinoxaline, triazole, isoquinoline, carbazole, indole, isoindole, oxazole and thiazole. More preferably, Z1, Z2 and Z4 independently represent groups selected from optionally substituted pyridin-2-yl, optionally substituted imidazol-2-yl, optionally substituted imidazol-4-yl, optionally substituted pyrazol-1-yl, and optionally substituted quinolin-2-yl. Most preferred is that Z1, Z2 and Z4 each represent optionally substituted pyridin-2-yl.

The groups Z1, Z2 and Z4 if substituted, are preferably substituted by a group selected from C₁₋₄-alkyl, aryl, arylalkyl, heteroaryl, methoxy, hydroxy, nitro, amino, carboxyl, halo, and carbonyl. Preferred is that Z1, Z2 and Z4 are each substituted by a methyl group. Also, we prefer that the Z1 groups represent identical groups.

The groups R5, R6, R7, R8 preferably independently represent a group selected from -H, hydroxy-C₀-C₂₀-alkyl, halo-C₀-C₂₀-alkyl, nitroso, formyl-C₀-C₂₀-alkyl, carboxyl-C₀-C₂₀-alkyl and esters and salts thereof, carbamoyl-C₀-C₂₀-alkyl, sulfo-C₀-C₂₀-alkyl and esters and salts thereof, sulfamoyl-C₀-C₂₀-alkyl, amino-C₀-C₂₀-alkyl, aryl-C₀-C₂₀-alkyl, C₀-C₂₀-alkyl, alkoxy-C₀-C₈-alkyl, carbonyl-C₀-C₆-alkoxy, and C₀-C₂₀-alkylamide.

Each Q1 preferably represents a covalent bond or C1-C4-alkylene, more preferably a covalent bond, methylene or ethylene, most preferably a covalent bond.

Group Q preferably represents, a covalent bond or C1-C4-alkylene, more preferably a covalent bond.

Non-coordinated group T preferably represents hydrogen, hydroxy, methyl, ethyl, benzyl, or methoxy.

In one aspect of the present invention the group U in formula (I) represents a coordinating group of the general formula (II):

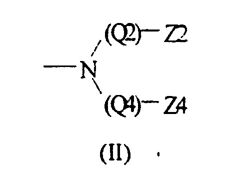

According to this aspect, it is preferred that Z2 represents an optionally substituted heterocyclic ring or an optionally substituted heteroaromatic ring selected from pyridine, pyrimidine, pyrazine, pyrazole, imidazole, benzimidazole, quinoline, quinoxaline, triazole, isoquinoline, carbazole, indole, isoindole, oxazole and thiazole, more preferably optionally substituted pyridin-2-yl or optionally substituted benzimidazol-2-yl.

It is also preferred, in this aspect, that Z4 represents an optionally substituted heterocyclic ring or an optionally substituted heteroaromatic ring selected from pyridine, pyrimidine, pyrazine, pyrazole, imidazole, benzimidazole, quinoline, quinoxaline, triazole, isoquinoline, carbazole, indole, isoindole, oxazole and thiazole, more preferably optionally substituted pyridin-2-yl, or an non-coordinating group selected from hydrogen, hydroxy, alkoxy, alkyl, alkenyl, cycloalkyl, aryl, or benzyl.

In preferred embodiments of this aspect, the ligand L is selected from: 1,1-bis(pyridin-2-yl)-N-methyl-N-(pyridin-2-ylmethyl)methylamine 1,1-bis(pyridin-2-yl)-N,N-bis(6-methyl-pyridin-2-ylmethyl)methylamine 1,1-bis(pyridin-2-yl)-N,N-bis(5-carboxymethyl-pyridin-2-ylmethyl)methylamine 1,1-bis(pyridin-2-yl)-1-benzyl-N,N-bis(pyridin-2-ylmethyl)methylamine 1,1-bis(pyridin-2yl)-N,N-bis(benzimidazol-2-ylmethyl)methylamine

In a variant of this aspect, the group Z4 in formula (II) represents a group of the general formula (IIa):

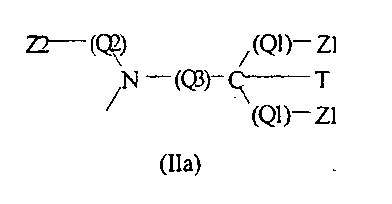

In this variant, Q4 preferably represents optionally substituted alkylene, preferably -CH₂-CHOH-CH₂- or -CH₂-CH₂-CH₂-. In a preferred embodiment of this variant, L represents the ligand: 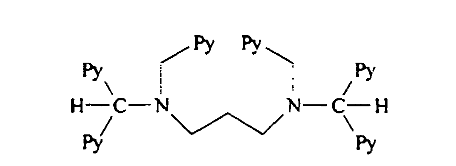 wherein -Py represents pyridin-2-yl.

In another aspect of the present invention, the group U in formula (I) represents a coordinating group of the general formula (III): 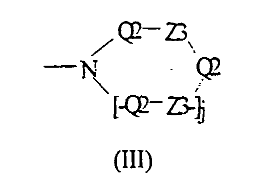 wherein j is 1 or 2, preferably 1.

According to this aspect, each Q2 preferably represents -(CH₂)ₙ- (n=2-4), and each Z3 preferably represents -N(R)- wherein R = -H or C₁₋₄-alkyl, preferably methyl.

In preferred embodiments of this aspect, L represents a ligand selected from: 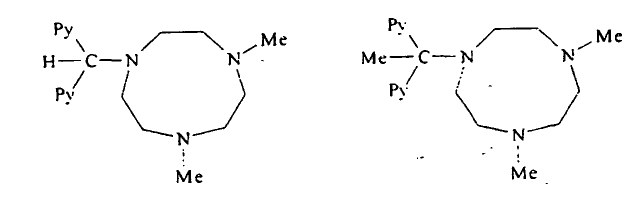 wherein -Py represents pyridin-2-yl.

In yet another aspect of the present invention, the group U in formula (I) represents a coordinating group of the general formula (IV):

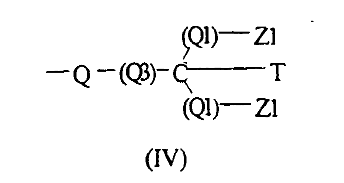

In this aspect, Q preferably represents -N(T)- (wherein T= -H, methyl, or benzyl) or pyridin-diyl.

In preferred embodiments of this aspect, L represents a ligand selected from: 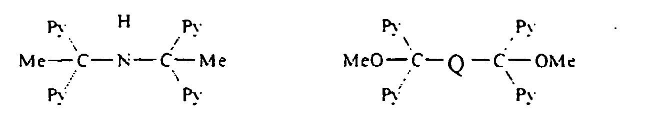 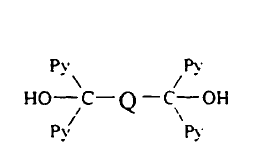 wherein -Py represents pyridin-2-yl, and -Q- represents pyridin-2,6-diyl.

In a further aspect, the present invention provides a ligand L as defined above, with the proviso that T in formula (I) is not benzyl.

In a further aspect, the present invention provides a complex (A1) as defined above, with the proviso that T in formula (I) is not benzyl.

In a further aspect, the present invention provides a ligand as defined above, with the proviso that U represents a non-coordinated group T or a coordinated group of the formula (II) or (III), and if U represents a coordinating group of formula (II) and Z1=Z2=Z4=unsubstituted pyridin-2-yl, T is not hydrogen, methyl or benzyl.

In preferred embodiments of this aspect, the ligand is selected from:
1,1-bis(pyridin-2-yl)-N-methyl-N(pyridin-2-ylmethyl)methylamine;
1,1-bis(pyridin-2-yl)-N,N-bis(6-methyl-pyridin-2-ylmethyl)methylamine;
1,1-bis(pyridin-2-yl)-N,N-bis(5-carboxymethyl-pyridin-2-yl)methylamine;
1,1-bis(pyridin-2yl)-N,N-bis(benzimidazol-2-ylmethyl)methylamine;
2,6-bis(pyridin-2ylmethyl)-1,1,7,7-tetrakis(pyridin-2-yl)-2,6-diazaheptane; or a ligand selected from: 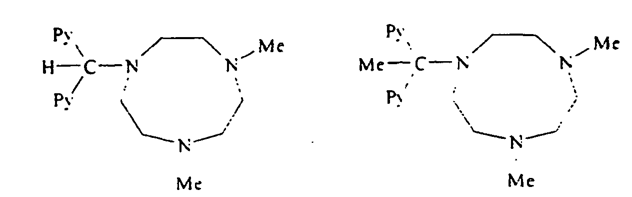 wherein -Py represents pyridin-2-yl.

In a further aspect, the present invention provides a transition-metal complex of the general formula (A1):

[MₐLₖXₙ]Yₘ (A1)

in which:
M represents a metal selected from Mn(II)-(III)-(IV)-(V), Cu(I)-(II)-(III), Fe(II)-(III)-(IV)-(V), Co(I)-(II)-(III), Ti(II)-(III)-(IV), V(II)-(III)-(IV)-(V), Mo(II)-(III)-(IV)-(V)-(VI) and W(IV)-(V)-(VI);
X represents a coordinating species selected from any mono, bi or tri charged anions and any neutral molecules able to coordinate the metal in a mono, bi or tridentate manner;
Y represents any non-coordinated counter ion;
a represents an integer from 1 to 10;
k represents an integer from 1 to 10;
n represents an integer from 1 to 10;
m represents zero or an integer from 1 to 20; and
L represents a ligand (or its protonated or deprotonated analogue) as defined above, with the proviso that U represents a non-coordinated group T or a coordinated group of the formula (II) or (III), and if U represents a coordinating group of formula (II) and Z1=Z2=Z4=unsubstituted pyridin-2-yl, T is not hydrogen, methyl or benzyl.

In preferred embodiments of this aspect, the ligand L is selected from:
1,1-bis(pyridin-2-yl)-N-methyl-N(pyridin-2-ylmethyl)methylamine;
1,1-bis(pyridin-2-yl)-N,N-bis(6-methyl-pyridin-2-ylmethyl)methylamine;
1,1-bis(pyridin-2-yl)-N,N-bis(5-carboxymethyl-pyridin-2-yl)methylamine;
1,1-bis(pyridin-2yl)-N,N-bis(benzimidazol-2-ylmethyl)methylamine;
2.6-bis(pyridin-2ylmethyl)-1,1,7,7-tetrakis(pyridin-2-yl)-2,6-diazaheptane; or a ligand selected from: 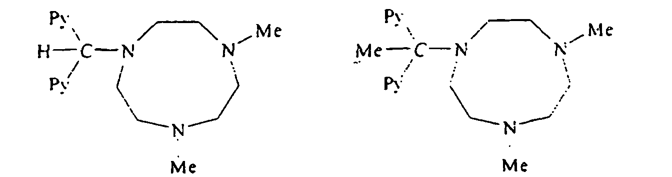 wherein -Py represents pyridin-2-yl.

Preferably, the metal ion in the complex of this aspect is selected from Fe(II)-(III)-(IV). Mn(II)-(III)-(IV)-(V), Cu(I)-(II)-(III) and Co(I)-(II)-(III), more preferably Fe(II)-(III) or Mn(II)-(III)-(IV).

The counter ions Y in formula (A1) balance the charge z on the complex formed by the ligand L, metal M and coordinating species X. Thus, if the charge z is positive, Y may be an anion such as RCOO⁻, BPh₄⁻, ClO₄⁻, BF₄⁻, PF₆⁻, RSO₃⁻, RSO₄⁻, SO₄²⁻, NO₃⁻, F⁻, Cl⁻, Br⁻, or I⁻, with R being hydrogen, optionally substituted alkyl or optionally substituted aryl. If z is negative, Y may be a common cation such as an alkali metal, alkaline earth metal or (alkyl)ammonium cation.

Suitable counter ions Y include those which give rise to the formation of storage-stable solids. Preferred counter ions for the preferred metal complexes are selected from R⁷COO⁻, ClO₄⁻, BF₄⁻, PF₆⁻, RSO₃⁻ (in particular CF₃SO₃⁻), RSO₄⁻, SO₄²⁻ , NO₃⁻, F⁻, Cl⁻, Br⁻, and I⁻, wherein R represents hydrogen or optionally substituted phenyl, naphthyl or C₁-C₄ alkyl.

It will be appreciated that the complex (A1) can be formed by any appropriate means, including *in situ* formation whereby precursors of the complex are transformed into the active complex of general formula (A1) under conditions of storage or use. Preferably, the complex is formed as a well-defined complex or in a solvent mixture comprising a salt of the metal M and the ligand L or ligand L-generating species. Alternatively, the catalyst may be formed *in situ* from suitable precursors for the complex, for example in a solution or dispersion containing the precursor materials. In one such example, the active catalyst may be formed *in situ* in a mixture comprising a salt of the metal M and the ligand L, or a ligand L-generating species, in a suitable solvent. Thus, for example, if M is iron, an iron salt such as FeSO₄ can be mixed in solution with the ligand L, or a ligand L-generating species, to form the active complex. Thus, for example, the composition may formed from a mixture of the ligand L and a metal salt MXₙ in which preferably n=1-5, more preferably 1-3. In another such example, the ligand L, or a ligand L-generating species, can be mixed with metal M ions present in the substrate or wash liquor to form the active catalyst *in situ*. Suitable ligand L-generating species include metal-free compounds or metal coordination complexes that comprise the ligand L and can be substituted by metal M ions to form the active complex according the formula (A1).

The bleaching compositions according to the present invention may be used for laundry cleaning, hard surface cleaning (including cleaning of lavatories, kitchen work surfaces, floors, mechanical ware washing etc.). As is generally known in the art, bleaching compositions are also employed in waste-water treatment, pulp bleaching during the manufacture of paper, leather manufacture, dye transfer inhibition, food processing, starch bleaching, sterilisation, whitening in oral hygiene preparations and/or contact lens disinfection.

In the context of the present invention bleaching should be understood as relating generally to the decolourisation of stains or of other materials attached to or associated with a substrate. However, it is envisaged that the present invention can be applied where a requirement is the removal and/or neutralisation by an oxidative bleaching reaction of malodours or other undesirable components attached to or otherwise associated with a substrate. Furthermore, in the context of the present invention bleaching is to be understood as being restricted to any bleaching mechanism or process that does not require the presence of light or activation by light. Thus, photobleaching compositions and processes relying on the use of photobleach catalysts or photobleach activators and the presence of light are excluded from the present invention.

In typical washing compositions the level of the catalyst is such that the in-use level is from 0.05µM to 50mM, with preferred in-use levels for domestic laundry operations falling in the range 0.5 µM to 100 µM, more preferably from 1 µM to 10 µM. Higher levels may be desired and applied in industrial bleaching processes, such as textile and paper pulp bleaching.

Preferably, the aqueous medium has a pH in the range from pH 6 to 13, more preferably from pH 6 to 11, still more preferably from pH 8 to 11, and most preferably from pH 8 to 10, in particular from pH 9 to 10.

The bleaching composition of the present invention has particular application in detergent formulations, especially for laundry cleaning. Accordingly, in another preferred embodiment, the present invention provides a detergent bleach composition comprising a bleaching composition as defined above and additionally a surface-active material, optionally together with detergency builder.

The bleach composition according to the present invention may for example contain a surface-active material in an amount of from 10 to 50% by weight. The surface-active material may be naturally derived, such as soap, or a synthetic material selected from anionic, nonionic, amphoteric, zwitterionic, cationic actives and mixtures thereof. Many suitable actives are commercially available and are fully described in the literature, for example in "Surface Active Agents and Detergents", Volumes I and II, by Schwartz, Perry and Berch.

Typical synthetic anionic surface-actives are usually water-soluble alkali metal salts of organic sulfates and sulfonates having alkyl groups containing from about 8 to about 22 carbon atoms, the term "alkyl" being used to include the alkyl portion of higher aryl groups. Examples of suitable synthetic anionic detergent compounds are sodium and ammonium alkyl sulfates, especially those obtained by sulfating higher (C₈-C₁₈) alcohols produced, for example, from tallow or coconut oil; sodium and ammonium alkyl (C₉-C₂₀) benzene sulfonates, particularly sodium linear secondary alkyl (C₁₀-C₁₅) benzene sulfonates; sodium alkyl glyceryl ether sulfates, especially those ethers of the higher alcohols derived from tallow or coconut oil fatty acid monoglyceride sulfates and sulfonates; sodium and ammonium salts of sulfuric acid esters of higher (C₉-C₁₈) fatty alcohol alkylene oxide, particularly ethylene oxide, reaction products; the reaction products of fatty acids such as coconut fatty acids esterified with isethionic acid and neutralised with sodium hydroxide: sodium and ammonium salts of fatty acid amides of methyl taurine; alkane monosulfonates such as those derived by reacting alpha-olefins (C₈-C₂₀) with sodium bisulfite and those derived by reacting paraffins with SO₂ and Cl₂ and then hydrolysing with a base to produce a random sulfonate; sodium and ammonium (C₇-C₁₂) dialkyl sulfosuccinates; and olefin sulfonates, which term is used to describe material made by reacting olefins, particularly (C₁₀-C₂₀) alpha-olefins, with SO₃ and then neutralising and hydrolysing the reaction product. The preferred anionic detergent compounds are sodium (C₁₀-C₁₅) alkylbenzene sulfonates, and sodium (C₁₆-C₁₈) alkyl ether sulfates.

Examples of suitable nonionic surface-active compounds which may be used, preferably together with the anionic surface-active compounds, include, in particular, the reaction products of alkylene oxides, usually ethylene oxide, with alkyl (C₆-C₂₂) phenols, generally 5-25 EO, *i.e*. 5-25 units of ethylene oxides per molecule; and the condensation products of aliphatic (C₈-C₁₈) primary or secondary linear or branched alcohols with ethylene oxide, generally 2-30 EO. Other so-called nonionic surface-actives include alkyl polyglycosides, sugar esters, long-chain tertiary amine oxides, long-chain tertiary phosphine oxides and dialkyl sulfoxides.

Amphoteric or zwitterionic surface-active compounds can also be used in the compositions of the invention but this is not normally desired owing to their relatively high cost. If any amphoteric or zwitterionic detergent compounds are used, it is generally in small amounts in compositions based on the much more commonly used synthetic anionic and nonionic actives.

The detergent bleach composition of the invention will preferably comprise from 1 to 15 % wt of anionic surfactant and from 10 to 40 % by weight of nonionic surfactant. In a further preferred embodiment, the detergent active system is free from C₁₆-C₁₂ fatty acid soaps.

The bleach composition of the present invention may also contains a detergency builder, for example in an amount of from about 5 to 80 % by weight, preferably from about 10 to 60 % by weight.

Builder materials may be selected from 1) calcium sequestrant materials. 2) precipitating materials, 3) calcium ion-exchange materials and 4) mixtures thereof.

Examples of calcium sequestrant builder materials include alkali metal polyphosphates, such as sodium tripolyphosphate; nitrilotriacetic acid and its water-soluble salts; the alkali metal salts of carboxymethyloxy succinic acid, ethylene diamine tetraacetic acid, oxydisuccinic acid, mellitic acid, benzene polycarboxylic acids, citric acid; and polyacetal carboxylates as disclosed in US-A-4,144,226 and US-A-4,146,495.

Examples of precipitating builder materials include sodium orthophosphate and sodium carbonate.

Examples of calcium ion-exchange builder materials include the various types of water-insoluble crystalline or amorphous aluminosilicates, of which zeolites are the best known representatives, *e.g*. zeolite A, zeolite B (also known as zeolite P), zeolite C, zeolite X, zeolite Y and also the zeolite P-type as described in EP-A-0,384,070.

In particular, the compositions of the invention may contain any one of the organic and inorganic builder materials, though, for environmental reasons, phosphate builders are preferably omitted or only used in very small amounts. Typical builders usable in the present invention are, for example, sodium carbonate, calcite/carbonate, the sodium salt of nitrilotriacetic acid, sodium citrate, carboxymethyloxy malonate, carboxymethyloxy succinate and water-insoluble crystalline or amorphous aluminosilicate builder materials, each of which can be used as the main builder, either alone or in admixture with minor amounts of other builders or polymers as co-builder.

It is preferred that the composition contains not more than 5% by weight of a carbonate builder, expressed as sodium carbonate, more preferably not more than 2.5 % by weight to substantially nil, if the composition pH lies in the lower alkaline region of up to 10.

Apan from the components already mentioned, the bleach composition of the present invention can contain any of the conventional additives in amounts of which such materials arc normally employed in fabric washing detergent compositions. Examples of these additives include buffers such as carbonates, lather boosters, such as alkanolamides, particularly the monoethanol amides derived from palmkernel fatty acids and coconut fatty acids; lather depressants, such as alkyl phosphates and silicones; antiredeposition agents, such as sodium carboxymethyl cellulose and alkyl or substituted alkyl cellulose ethers; stabilisers, such as phosphonic acid derivatives (*i.e*. Dequest® types); fabric softening agents; inorganic salts and alkaline buffering agents, such as sodium sulfate and sodium silicate; and, usually in very small amounts, fluorescent agents; perfumes; enzymes, such as proteases, cellulases, lipases, amylases and oxidases; germicides and colourants.

Transition metal sequestrants such as EDTA, and phosphonic acid derivatives such as EDTMP (ethylene diamine tetra(methylene phosphonate)) may also be included, in addition to the ligand specified, for example to improve the stability sensitive ingredients such as enzymes, fluorescent agents and perfumes, but provided the composition remains bleaching effective. However, the composition according to the present invention containing the ligand, is preferably substantially, and more preferably completely, devoid of transition metal sequestrants (other than the ligand).

Whilst the present invention is based on the catalytic bleaching of a substrate by atmospheric oxygen or air, it will be appreciated that small amounts of hydrogen peroxide or peroxy-based or -generating systems may be included in the composition, if desired. Therefore, by "substantially devoid of peroxygen bleach or peroxy-based or - generating bleach systems" is meant that the composition contains from 0 to 50 %, preferably from 0 to 10 %, more preferably from 0 to 5 %, and optimally from 0 to 2 % by molar weight on an oxygen basis, of peroxygen bleach or peroxy-based or - generating bleach systems. Preferably, however, the composition will be wholly devoid of peroxygen bleach or peroxy-based or -generating bleach systems.

Thus, at least 10 %, preferably at least 50 % and optimally at least 90 % of any bleaching of the substrate is effected by oxygen sourced from the air.

According to the fourth aspect, the catalyst may be contacted to the textile fabric in any suitable manner. For example, it may be applied in dry form, such as in powder form, or in a liquor that is then dried, for example as an aqueous spray-on fabric treatment fluid or a wash liquor for laundry cleaning, or a non-aqueous dry cleaning fluid or spray-on aerosol fluid. Other suitable means of contacting the catalyst to the textile may be used, as further explained below.

Any suitable textile that is susceptible to bleaching or one that one might wish to subject to bleaching may be used. Preferably the textile is a laundry fabric or garment.

The bleaching method of the fourth aspect may be carried out by simply leaving the substrate in contact with the catalyst for a sufficient period of time. Preferably, however, the catalyst is in an aqueous medium, and the aqueous medium on or containing the substrate is agitated.

In a preferred embodiment, the treated textile is dried, by allowing it to dry under ambient temperature or at elevated temperatures.

In a particularly preferred embodiment the method according to the fourth aspect is carried out on a laundry fabric using aqueous treatment liquor. In particular the treatment may be effected in, or as an adjunct to, an essentially conventional wash cycle for cleaning laundry. More preferably, the treatment is carried out in an aqueous detergent wash liquor. The catalyst can be delivered into the wash liquor from a powder, granule, pellet, tablet, block, bar or other such solid form. The solid form can comprise a carrier, which can be particulate, sheet-like or comprise a three-dimensional object. The carrier can be dispersible or soluble in the wash liquor or may remain substantially intact. In other embodiments, the catalyst can be delivered into the wash liquor from a paste, gel or liquid concentrate.

It is particularly advantageous that the catalyst used in the method of the fourth aspect makes use of atmospheric oxygen in its bleaching activity. This avoids the requirement that peroxygen bleaches and/or other relatively large quantities of reactive substances need be used in the treatment process. Consequently, only a relatively small quantity of bleach active substance need be employed and this allows dosage routes to be exploited which could previously not be used. Thus, while it is preferable to include the catalyst in a composition that is normally used in a washing process, such as a pre-treatment, main-wash, conditioning composition or ironing aid, other means for ensuring that the catalyst is present in the wash liquor may be envisaged.

For example, it is envisaged that the catalyst can be presented in the form of a body from which it is slowly released during the whole or part of the laundry process. Such release can occur over the course of a single wash or over the course of a plurality of washes. In the latter case it is envisaged that the catalyst can be released from a carrier substrate used in association with the wash process, e.g. from a body placed in the dispenser drawer of a washing machine, elsewhere in the delivery system or in the drum of the washing machine. When used in the drum of the washing machine the carrier can be freely moving or fixed relative to the drum. Such fixing can be achieved by mechanical means, for example by barbs that interact with the drum wall, or employ other forces, for example a magnetic force. The modification of a washing machine to provide for means to hold and retain such a carrier is envisaged similar means being known from the analogous art of toilet block manufacture. Freely moving carriers such as shuttles for dosage of surfactant materials and/or other detergent ingredients into the wash can comprise means for the release of the catalyst into the wash.

In the alternative, the catalyst can be presented in the form of a wash additive that preferably is soluble. The additive can take any of the physical forms used for wash additives, including powder, granule, pellet, sheet, tablet, block, bar or other such solid form or take the form of a paste, gel or liquid. Dosage of the additive can be unitary or in a quantity determined by the user. While it is envisaged that such additives can be used in the main washing cycle, the use of them in the conditioning or drying cycle is not hereby excluded.

The present invention is not limited to those circumstances in which a washing machine is employed, but can be applied where washing is performed in some alternative vessel. In these circumstances it is envisaged that the catalyst can be delivered by means of slow release from the bowl, bucket or other vessel which is being employed, or from any implement which is being employed, such as a brush, bat or dolly, or from any suitable applicator.

Suitable pre-treatment means for application of the catalyst to the textile material prior to the main wash include sprays, pens, roller-ball devices, bars, soft solid applicator sticks and impregnated cloths or cloths containing microcapsules. Such means are well known in the analogous art of deodorant application and/or in spot treatment of textiles. Similar means for application are employed in those embodiments where the catalyst is applied after the main washing and/or conditioning steps have been performed, e.g. prior to or after ironing or drying of the cloth. For example, the catalyst may be applied using tapes, sheets or sticking plasters coated or impregnated with the substance, or containing microcapsules of the substance. The catalyst may for example be incorporated into a drier sheet so as to be activated or released during a tumble-drier cycle, or the substance can be provided in an impregnated or microcapsule-containing sheet so as to be delivered to the textile when ironed.

Throughout the description and claims generic groups have been used, for example alkyl, alkoxy, aryl. Unless otherwise specified the following are preferred group restrictions that may be applied to generic groups found within compounds disclosed herein:
alkyl: linear and branched C1-C8-alkyl,
alkenyl: C2-C6-alkenyl,
cycloalkyl: C3-C8-cycloalkyl,
alkoxy: C1-C6-alkoxy,
alkylene: selected from the group consisting of: methylene; 1,1-ethylene; 1,2-ethylene; 1,1-propylidene; 1,2-propylene; 1,3-propylene; 2,2-propylidene; butan-2-ol-1,4-diyl; propan-2-ol-1,3-diyl; 1,4-butylene; cyclohexane-1,1-diyl; cyclohexan-1,2-diyl; cyclohexan-1,3-diyl; cyclohexan-1,4-diyl; cyclopentane-1,1-diyl; cyclopentan-1,2-diyl; and cyclopentan-1,3-diyl,
aryl: selected from homoaromatic compounds having a molecular weight under 300,
arylene: selected from the group consisting of: 1,2-phenylene; 1,3-phenylene; 1,4-phenylene; 1,2-naphtalenylene; 1,3-naphtalenylene; 1,4-naphtalenylene; 2,3-naphtalenylene; 1-hydroxy-2,3-phenylene; 1-hydroxy-2,4-phenylene; 1-hydroxy-2,5-phenylene; and 1-hydroxy-2,6-phenylene,
heteroaryl: selected from the group consisting of: pyridinyl; pyrimidinyl; pyrazinyl; triazolyl; pyridazinyl; 1,3,5-triazinyl; quinolinyl; isoquinolinyl; quinoxalinyl; imidazolyl; pyrazolyl; benzimidazolyl; thiazolyl; oxazolidinyl; pyrrolyl; carbazoyl; indolyl; and isoindolyl, wherein the heteroaryl may be connected to the compound via any atom in the ring of the selected heteroaryl,
heteroarylene: selected from the group consisting of: pyridindiyl; quinolindiyl; pyrazodiyl; pyrazoldiyl; triazolediyl; pyrazindiyl; and imidazolediyl, wherein the heteroarylene acts as a bridge in the compound via any atom in the ring of the selected heteroarylene, more specifically preferred are: pyridin-2,3-diyl; pyridin-2,4-diyl; pyridin-2,5-diyl; pyridin-2,6-diyl; pyridin-3,4-diyl; pyridin-3,5-diyl; quinolin-2,3-diyl; quinolin-2,4-diyl; quinolin-2,8-diyl; isoquinolin-1,3-diyl; isoquinolin-1,4-diyl; pyrazol-1,3-diyl; pyrazol-3,5-diyl; triazole-3,5-diyl; triazole-1,3-diyl; pyrazin-2,5-diyl; and imidazole-2,4-diyl,
heterocycloalkyl: selected from the group consisting of: pyrrolinyl; pyrrolidinyl; morpholinyl; piperidinyl; piperazinyl; hexamethylene imine; 1,4-piperazinyl; tetrahydrothiophenyl; tetrahydrofuranyl: 1,4,7-triazacyclononanyl; 1,4,8,11-tetraazacyclotetradecanyl; 1,4,7,10,13-pentaazacyclopentadecanyl; 1,4-diaza-7-thiacyclononanyl; 1,4-diaza-7-oxa-cyclononanyl; 1,4,7,10-tetraazacyclododecanyl; 1,4-dioxanyl; 1,4,7-trithia-cyclononanyl; tetrahydropyranyl; and oxazolidinyl, wherein the heterocycloalkyl may be connected to the compound via any atom in the ring of the selected heterocycloalkyl,
heterocycloalkylene: selected from the group consisting of: piperidin-1,2-ylene; piperidin-2,6-ylene; piperidin-4,4-ylidene; 1,4-piperazin-1,4-ylene; 1,4-piperazin-2,3-ylene; 1,4-piperazin-2,5-ylene; 1,4-piperazin-2,6-ylene; 1,4-piperazin-1,2-ylene; 1,4-piperazin-1,3-ylene; 1,4-piperazin-1,4-ylene; tetrahydrothiophen-2,5-ylene; tetrahydrothiophen-3,4-ylene; tetrahydrothiophen-2,3-ylene; tetrahydrofuran-2,5-ylene; tetrahydrofuran-3,4-ylene; tetrahydrofuran-2,3-ylene; pyrrolidin-2,5-ylene; pyrrolidin-3,4-ylene; pyrrolidin-2,3-ylene; pyrrolidin-1,2-ylene; pyrrolidin-1,3-ylene; pyrrolidin-2,2-ylidene; 1,4,7-triazacyclonon-1,4-ylene; 1,4,7-triazacyclonon-2,3-ylene; 1,4,7-triazacyclonon-2,9-ylene; 1,4,7-triazacyclonon-3,8-ylene; 1,4,7-triazacyclonon-2,2-ylidene; 1,4,8,11-tetraazacyclotetradec-1,4-ylene; 1,4,8,11-tetraazacyclotetradec-1,8-ylene; 1,4,8,11-tetraazacyclotetradec-2,3-ylene; 1,4,8,11-tetraazacyclotetradec-2,5-ylene; 1,4,8,11-tetraazacyclotetradec-1,2-ylene; 1,4,8,11-tetraazacyclotetradec-2,2-ylidene; 1,4,7,10-tetraazacyclododec-1,4-ylene; 1,4,7,10-tetraazacyclododec-1,7-ylene; 1,4,7,10-tetraazacyclododec-1,2-ylene; 1,4,7,10-tetraazacyclododec-2,3-ylene; 1,4,7,10-tetraazacyclododec-2,2-ylidene; 1,4,7,10,13-pentaazacyclopentadec-1,4-ylene; 1,4,7,10,13-pentaazacyclopentadec-1,7-ylene; 1,4,7,10,13-pentaazacyclopentadec-2,3-ylene; 1,4,7,10,13-pentaazacyclopentadec-1,2-ylene; 1,4,7,10,13-pentaazacyclopentadec-2,2-ylidene; 1,4-diaza-7-thia-cyclonon-1,4-ylene; 1,4-diaza-7-thia-cyclonon-1,2-ylene; 1,4-diaza-7-thia-cyclonon-2,3-ylene; 1,4-diaza-7-thia-cyclonon-6,8-ylene; 1,4-diaza-7-thia-cyclonon-2,2-ylidene; 1,4-diaza-7-oxa-cyclonon-1,4-ylene; 1,4-diaza-7-oxa-cyclonon-1,2-ylene; 1,4-diaza-7-oxa-cyclonon-2,3-ylene; 1,4-diaza-7-oxa-cyclonon-6,8-ylene; 1,4-diaza-7-oxa-cyclonon-2,2-ylidene; 1,4-dioxan-2.3-ylene; 1,4-dioxan-2,6-ylene; 1,4-dioxan-2,2-ylidene; tetrahydropyran-2,3-ylene; tetrahydropyran-2,6-ylene; tetrahydropyran-2,5-ylene; tetrahydropyran-2,2-ylidene; 1,4,7-trithia-cyclonon-2,3-ylene; 1,4,7-trithia-cyclonon-2,9-ylene; and 1,4,7-trithia-cyclonon-2,2-ylidene,
amine: the group -N(R)₂ wherein each R is independently selected from: hydrogen; C1-C6-alkyl; C1-C6-alkyl-C6H5; and phenyl, wherein when both R are C1-C6-alkyl both R together may form an -NC3 to an -NC5 heterocyclic ring with any remaining alkyl chain forming an alkyl substituent to the heterocyclic ring,
halogen: selected from the group consisting of: F; Cl; Br and I,
sulfonate: the group -S(O)₂OR, wherein R is selected from: hydrogen; C1-C6-alkyl; phenyl; C1-C6-alkyl-C6H5; Li; Na; K; Cs; Mg; and Ca,
sulfate:the group -OS(O)₂OR, wherein R is selected from: hydrogen; C1-C6-alkyl; phenyl; C1-C6-alkyl-C6H5; Li; Na; K; Cs; Mg; and Ca.
sulfone: the group -S(O)₂R, wherein R is selected from: hydrogen; C1-C6-alkyl; phenyl; C1-C6-alkyl-C6H5 and amine (to give sulfonamide) selected from the group: -NR'2, wherein each R' is independently selected from: hydrogen; C1-C6-alkyl; C1-C6-alkyl-C6H5; and phenyl, wherein when both R' are C1-C6-alkyl both R' together may form an -NC3 to an -NC5 heterocyclic ring with any remaining alkyl chain forming an alkyl substituent to the heterocyclic ring,
carboxylate derivative: the group -C(O)OR, wherein R is selected from: hydrogen; C1-C6-alkyl; phenyl; C1-C6-alkyl-C6H5; Li; Na; K; Cs; Mg; and Ca.
carbonyl derivative: the group -C(O)R, wherein R is selected from: hydrogen: C1-C6-alkyl; phenyl; C1-C6-alkyl-C6H5 and amine (to give amide) selected from the group: -NR'2. wherein each R' is independently selected from: hydrogen; C1-C6-alkyl; C1-C6-alkyl-C6H5; and phenyl, wherein when both R' are C1-C6-alkyl both R' together may form an -NC3 to an -NC5 heterocyclic ring with any remaining alkyl chain forming an alkyl substituent to the heterocyclic ring,
phosphonate: the group -P(O)(OR)₂, wherein each R is independently selected from: hydrogen; C1-C6-alkyl; phenyl; C1-C6-alkyl-C6H5; Li; Na; K; Cs; Mg; and Ca,
phosphate: the group -OP(O)(OR)₂, wherein each R is independently selected from: hydrogen; C1-C6-alkyl; phenyl; C1-C6-alkyl-C6H5; Li; Na; K; Cs; Mg; and Ca.
phosphine: the group -P(R)₂, wherein each R is independently selected from: hydrogen; C1-C6-alkyl; phenyl; and C1-C6-alkyl-C6H5,
phosphine oxide: the group -P(O)R₂, wherein R is independently selected from: hydrogen; C1-C6-alkyl; phenyl; and C1-C6-alkyl-C6H5; and amine (to give phosphonamidate) selected from the group: -NR'2, wherein each R' is independently selected from: hydrogen; C1-C6-alkyl; C1-C6-alkyl-C6H5; and phenyl, wherein when both R' are C1-C6-alkyl both R' together may form an -NC3 to an -NC5 heterocyclic ring with any remaining alkyl chain forming an alkyl substituent to the heterocyclic ring.

Unless otherwise specified the following are more preferred group restrictions that may be applied to groups found within compounds disclosed herein:
alkyl: linear and branched C1-C6-alkyl,
alkenyl: C3-C6-alkenyl,
cycloalkyl: C6-C8-cycloalkyl,
alkoxy: C1-C4-alkoxy,
alkylene: selected from the group consisting of: methylene; 1,2-ethylene; 1,3-propylene; butan-2-ol-1,4-diyl; 1,4-butylene; cyclohexane-1,1-diyl; cyclohexan-1,2-diyl; cyclohexan-1,4-diyl; cyclopentane-1,1-diyl; and cyclopentan-1,2-diyl,
aryl: selected from group consisting of: phenyl; biphenyl; naphthalenyl; anthracenyl; and phenanthrenyl,
arylene: selected from the group consisting of: 1,2-phenylene; 1,3-phenylene; 1,4-phenylene; 1,2-naphtalenylene; 1,4-naphtalenylene; 2,3-naphtalenylene and 1-hydroxy-2,6-phenylene,
heteroaryl: selected from the group consisting of: pyridinyl; pyrimidinyl; quinolinyl; pyrazolyl; triazolyl; isoquinolinyl; imidazolyl; and oxazolidinyl, wherein the heteroaryl may be connected to the compound via any atom in the ring of the selected heteroaryl,
heteroarylene: selected from the group consisting of: pyridin-2,3-diyl; pyridin-2,4-diyl; pyridin-2,6-diyl; pyridin-3,5-diyl; quinolin-2,3-diyl; quinolin-2,4-diyl; isoquinolin-1,3-diyl; isoquinolin-1,4-diyl; pyrazol-3,5-diyl; and imidazole-2,4-diyl,
heterocycloalkyl; selected from the group consisting of: pyrrolidinyl; morpholinyl; piperidinyl; piperidinyl; 1,4-piperazinyl; tetrahydrofuranyl; 1,4,7-triazacyclononanyl; 1,4,8,11-tetraazacyclotetradecanyl; 1,4,7,10,13-pentaazacyclopentadecanyl; 1,4,7,10-tetraazacyclododecanyl; and piperazinyl, wherein the heterocycloalkyl may be connected to the compound via any atom in the ring of the selected heterocycloalkyl,
heterocycloalkylene; selected from the group consisting of: piperidin-2,6-ylene; piperidin-4,4-ylidene; 1,4-piperazin-1,4-ylene; 1,4-piperazin-2,3-ylene; 1,4-piperazin-2,6-ylene; tetrahydrothiophen-2,5-ylene; tetrahydrothiophen-3,4-ylene; tetrahydrofuran-2.5-ylene; tetrahydrofuran-3,4-ylene; pyrrolidin-2,5-ylene; pyrrolidin-2,2-ylidene; 1,4,7-triazacyclonon-1,4-ylene; 1,4,7-triazacyclonon-2,3-ylene; 1,4,7-triazacyclonon-2,2-ylidene; 1,4,8,11-tetraazacyclotetradec-1,4-ylene; 1,4,8,11-tetraazacyclotetradec-1,8-ylene; 1,4,8,11-tetraazacyclotetradec-2,3-ylene; 1,4,8,11-tetraazacyclotetradec-2,2-ylidene; 1,4,7,10-tetraazacyclododec-1,4-ylene; 1,4,7,10-tetraazacyclododec-1,7-ylene; 1,4,7,10-tetraazacyclododec-2,3-ylene; 1,4,7,10-tetraazacyclododec-2,2-ylidene; 1,4,7,10,13-pentaazacyclopentadec-1,4-ylene; 1,4,7,10,13-pentaazacyclopentadec-1,7-ylene; 1,4-diaza-7-thia-cyclonon-1,4-ylene; 1,4-diaza-7-thia-cyclonon-2,3-ylene; 1,4-diaza-7-thia-cyclonon-2,2-ylidene; 1,4-diaza-7-oxa-cyclonon-1,4-ylene; 1,4-diaza-7-oxa-cyclonon-2,3-ylene; 1,4-diaza-7-oxa-cyclonon-2,2-ylidene; 1,4-dioxan-2,6-ylene; 1,4-dioxan-2,2-ylidene; tetrahydropyran-2,6-ylene; tetrahydropyran-2,5-ylene; and tetrahydropyran-2,2-ylidene,
amine: the group -N(R)₂, wherein each R is independently selected from: hydrogen; C1-C6-alkyl; and benzyl,
halogen: selected from the group consisting of: F and Cl,
sulfonate: the group -S(O)₂OR, wherein R is selected from: hydrogen; C1-C6-alkyl; Na; K; Mg; and Ca,
sulfate:the group -OS(O)₂OR, wherein R is selected from: hydrogen; C1-C6-alkyl; Na; K; Mg; and Ca.
sulfone: the group -S(O)₂R, wherein R is selected from: hydrogen; C1-C6-alkyl; benzyl and amine selected from the group: -NR'2, wherein each R' is independently selected from: hydrogen; C1-C6-alkyl; and benzyl,
carboxylate derivative: the group -C(O)OR, wherein R is selected from hydrogen; Na; K; Mg; Ca; C1-C6-alkyl; and benzyl,
carbonyl derivative: the group: -C(O)R, wherein R is selected from: hydrogen; C1-C6-alkyl; benzyl and amine selected from the group: -NR'2, wherein each R' is independently selected from: hydrogen; C1-C6-alkyl; and benzyl,
phosphonate: the group -P(O)(OR)₂, wherein each R is independently selected from: hydrogen; C1-C6-alkyl; benzyl; Na; K; Mg; and Ca.
phosphate: the group -OP(O)(OR)₂, wherein each R is independently selected from: hydrogen; C1-C6-alkyl; benzyl; Na; K; Mg; and Ca,
phosphine: the group -P(R)₂, wherein each R is independently selected from: hydrogen; C1-C6-alkyl; and benzyl,
phosphine oxide: the group -P(O)R₂, wherein R is independently selected from: hydrogen; C1-C6-alkyl; benzyl and amine selected from the group: -NR'2, wherein each R' is independently selected from: hydrogen; C1-C6-alkyl; and benzyl.

The invention will now be further illustrated by way of the following non-limiting examples:

### EXAMPLES

The following compounds were prepared and tested for catalytic bleaching activity using air:
Compound **1**: [Fe(L¹)(CH₃CN)](ClO₄)₂
   L¹= 1,1-bis(pyridin-2-yl)-N-methyl-N-(pyridin-2-ylmethyl)methylamine
Compound **2**: [Fe(L²)(CH₃CN)](ClO₄)₂
   L²= 1,1-bis(pyridin-2-yl)-N,N-bis(6-methyl-pyridin-2-ylmethyl)methylamine
Compound **3**: [Fe₂(L³)(CH₃CN)₂](ClO₄)₄
   L³= 2,6-bis(pyridin-2-ylmethyl)-1,1,7,7-tetrakis(pyridin-2-yl)-2,6-diazaheptane
Compound **4**: [Fe(L⁴)(CH₃CN)](ClO₄)₂
   L⁴= 1,1-bis(pyridin-2-yl)-1-benzyl-N,N-bis(pyridin-2-ylmethyl)methylamine
Compound **5**: [Fe(L⁵)(CH₃CN)]ClO₄)₂
   L⁵= 1,1-bis(pyridin-2-yl)-N,N-bis(5-methoxycarbonyl-pyridin-2-ylmethyl)methylamine
Compound **6**: [Fe(L⁶)(CH₃CN)₂](ClO₄)₂
   L⁶= 1-(α,α-bis(pyridin-2-yl))methyl-4,7-dimethyl-1,4,7-triazacyclononane
Compound **7**: [Fe(L⁷)(CH₃CN)₂](ClO₄)₂
   L⁷= 1-(α,α-bis(pyridin-2-yl))ethyl-4,7-dimethyl-1,4,7-triazacyclononane
Compound **8**: 2,2,4,4-tetrakis(pyridin-2-yl)-3-azapentane (= L⁸) + Fe(ClO₄)₂
Compound **9**: L⁸ + Mn(ClO₄)₂.6H₂O
Compound **10**: L⁸ + Co(ClO₄)₂.6H₂O
Compound **11**:
   1,1-bis(pyridin-2yl)-N,N-bis(benzimidazol-2-yl-methyl)methylamine (= L⁹)
Compound **12**: L⁹ + Fe(ClO₄)₂.6H₂O
Compound **13**: L⁹ + Mn(ClO₄)₂.6H₂O
Compound **14**: L⁹ + Co(ClO₄)₂.6H₂O
Compound **15**: L⁹ + Cu(ClO₄)₂.6H₂O
Compound **16**: 2,6-bis(methoxy-bis(pyridin-2-yl)methyl)pyridin (=L¹⁰) + Co(ClO₄)₂.6H₂O
Compound **17**: 2,6-bis(hydroxy-bis-pyridin-2-yl)-methyl)pyridin (=L¹¹) + Co(ClO₄)₂.6H₂O

### Syntheses of compounds:

### Compound 1

### 1,1-bis(pyridin-2-yl)-N-methyl-N-(pyridin-2-ylmethyl)methylamine (L1) (=N-[di(2-pyridinyl)methyl]-N-methyl-N-(2-pyridinylmethyl)amine)

To di-2-pyridyl methyl amine (1.5 g, 8.1 mmol) was added freshly distilled pyridine-2-carboxaldehyde (900 mg, 8.4 mmol). After shaking the flask, the mixture was allowed to stand for approximately 2 hours. The white solid was collected and washed with cyclohexane to remove traces of unreacted starting material to give pure *N*-[di(2-pyridinyl)methyl]-*N*-[(*Z*)-2-pyridinylmethylidene]amine (2.02 g, 91 %). ¹H-NMR (CDCl₃, 300 MHz) δ 6.0 (s, 1H), 7.16 (m, 2H), 7.31 (m, 1H), 7.59 (m, 5H), 8.18 (m, 1H), 8.61 (m, 3H), 8.65 (s, 1H); MS (CI): m/z 275 (M+1).

To a solution of *N*-[di(2-pyridinyl)methyl]-*N*-[(*Z*)-2-pyridinylmethylidene]amine (1.5 g, 5.5 mmol) in methanol (20 ml) was added NaBH₄ (0.45 g, 11.8 mmol) in small portions. After stirring at room temperature for 2 hours HCl (aq) is added until the pH<2. After stirring for 30 min 5 N NaOH (aq) is added until the pH>9. The methanol is removed through evaporation and the aqueous layer is extracted with ethyl acetate (3 ×0 ml). The combined ethyl acetate layers are washed with brine (30 ml) and dried (Na₂SO₄). Evaporation of the solvent gave N-[di(2-pyridinyl)methyl]-N-(2-pyridinylmethyl)amine (N3Py) (1.35 g, 89 %) as a yellow oil. ¹H-NMR (CDCl₃, 300 MHz) δ 3.85 (s, 2H), 5.10 (s, 1 H), 7.03 (m, 3H), 7.41 (m, 6H), 8.46 (m, 3H); ¹³C NMR (CDCl₃, 50 MHz) δ 53.1 (t), 68.9 (d), 121.8 (d), 122.1 (d), 122.2 (d), 122.3 (d), 136.3 (d), 136.5 (d), 149.2 (d), 159.6 (s), 161.2 (s); MS (CI): m/z 277 (M+1).

To a solution of *N*-[di(2-pyridinyl)methyl]-*N*-(2-pyridinylmethyl)amine (1.262 g, 4.59 mmol) in 1,2-dichloroethane (35 ml) was added formaldehyde (37 % solution in water, 0.45 ml, 6.0 mmol). NaBH(OAc)₃ (3.92 g, 18.5 mmol) was added in small portions. After stirring for 7 h at room temperature saturated NaHCO_{3(aq)} (35 ml) was added and the 1,2-dichloroethane layer was separated. The aqueous layer is extracted with CH₂Cl₂ (3× 20 ml). The combined organic layers were washed with 1N NaOH (20 ml) and brine (20 ml), dried (Na₂SO₄) and the solvent removed *in vacuo* to give *N*-[di(2-pyridinyl)methyl]-*N*-methyl-*N*-(2-pyridinylmethyl)amine (1.235 g, 4.27 mmol, 93 %) as a slightly yellow oil. ¹H NMR (CDCl₃, 300 MHz) δ 2.19 (s, 3H), 3.72 (s, 2H), 4.96 (s, 1H), 7.14 (m, 3H), 7.71 (m, 6H), 8.56 (m, 3H); ¹³C NMR (CDCl₃, 50 MHz) δ 40.32 (q), 61.04 (t), 77.87 (d), 121.76 (d), 122.10 (d), 122.92 (d), 123.21 (d), 136.29 (d), 136.43 (d), 148.86 (d), 149.22 (d), 159.37 (s), 160.59 (s), MS (CI): m/z 291 (M+1).

### [(L1)Fe(CH₃CN)₂](ClO₄)₂

To a solution of *N*-[di(2-pyridinyl)methyl]-*N*-methyl-*N*-(2-pyidinylmethyl)amine (198 mg, 0.68 mmol) in acetonitrile (3 ml) was added a solution of Fe(ClO₄)₂·6H₂O (250 mg, 0.69 mmol) in methanol (3 ml). The solution was placed in an ethyl acetate bath and after 2 days [(L1)Fe(CH₃CN)₂](ClO₄)₂ (344 mg, 0.55 mmol, 81 %) was obtained as dark red crystals. ¹H NMR (CD₃CN, 300 MHz) δ 3.81 (br), 5.17 (br), 6.96 (br), 7.40 (t, J = 7.7 Hz), 7.64 (t, J = 7.7 Hz), 8.04 (t, J = 7.7 Hz), 8.59 (br), 8.70 (br), 8.87 (br), 9.02 (br), 11.26 (br), 11.40 (br); Anal. Calcd. for C₂₂H₂₄Cl₂FeN₆O₈: C 42.13, H 3.86, N 13.40; found: C 41.98, H 3.78, N 13.27.

### Compound 2

### 1,1-bis(pyridin-2-yl)-N,N-bis(6-methyl-pyridin-2-ylmethyl)methylamine,2HClO₄

To di-2-pyridyl methyl amine (1.8 g, 10 mmol) was mixed with 5 ml of 5N NaOH in water. To 2.83 g (20 mmol) of 2-picolylchloride (synthesised according to W. Mattes et al., Angew Chem., 75, 235 (1963)) was mixed with 5 ml of 5N NaOH in water. Both mixtures were colled in an ice bath and added together under stirring. Stirring was continued for 4 days at 20 °C. The reaction mixture was colled in an ice bath and under stirring 3 ml of 70% HClO₄ was added. The salt seperated as a liquid which became solid after scratching with a spatula. The yellow precipitate was washed 2 times 10 ml of water and 2 times of 5 ml of methanol. The compound was recrystallised from hot water and dried under vacuum over siccapent. Yield 3.1g (52.%); m.p. 168.5 °C, ¹H NMR (CD₃CN, 200 MHz) δ 2.88 (s) 6H; 4.21 (s) 4H; 5.91 (s) 1H; 7.33 (d) 2H; 7.43 (d) 2H; 7.63 (m) 4H; 7.99 (t) 2H; 8.15 (t) 2H; 8.82 (d) 2H, ¹³C-NMR: 25.80; 58.34; 75.00; 122.60; 129.00; 129.74; 130.82; 132.00; 145.00; 150.80; 153.80.
Anal. Calcd. for C₂₃H₂₃Cl₂N₅O₈: C 48.6, H 4.1, N 12.3, Cl 12.5%; found: C 48.5, H 4.7, N 11.5, Cl 11.5%.

The corresponding iron complex was synthesised as described for the non-methylated analogue (reference M. Lubben et al., **34**, 1512 (1995)).

### Compound 3

### 2,6-bis(pyridin-2-ylmethyl)-1,1,7,7-tetrakis(pyridin-2-yl)-2,6-diazaheptane (L3)

### Dipyridyl-methylchloride

To a solution of 9.2g of dipyridylketone in 200ml methanol was added 1g sodium borohydride in small portions over 0.5h. The reaction was exothermic. After completion of the addition the mix was stirred 15'. TLC analysis showed the conversion to be quantitive. To the mix was added 10ml of cencentrated hydrochloric acid and the acid solution was concentrated by evaporation in vacuo. Water was added and the acidic water phase is washed with dichloromethane. Now 100ml of 2N NaOH was added and the resulting alkaline mixture was extracted 3x with dichloromethane. The combined organic layers were dried over sodium sulfate, filtered and evaporated giving 9g of the dipyridylcarbinol. It was found that the compound degraded slowly and CDCl₃, samples were prepared just before measurement.
¹H NMR (CDCl₃) (ppm): 5.86 (s, 1H, Py2C-H); 7.15 (m, 2H, Py-H); 7.50 (m, 2H, Py-H); 7.62 (m, 2H, Py-H); 8.53 (m, 2H, Py-H)
¹³C NMR (CDCl3) (ppm): 76.3, 122.2, 123.6, 137.9, 149.1, 161.9

### N,N'-(Di-pyrid-2-yl methyl)-1,3-diaminopropane

2-Pyridine carboxaldehyde (9.0g, 84mmol) was added dropwise to a solution of 1,3-diaminopropane (3.0g, 40mmol) in methanol (100ml). The mixture became hot. After stirring for 30 min. NaBH₄ (4.0g, 105mmol) was added in portions. After addition of the first 0.9g, sodium tetraborate.10aq (7.0g, 18mmol) was added. When the addition was complete it was stirred at ambient temperature for 45 min then evaporated to approx. 50ml, water added (250ml) and extracted (x4) with CHCl3. The extracts were washed with saturated NaCl then dried and evaporated to leave a pale yellow oil (9.52g). This was short path distilled to give a forerun of 140mg, b.p. up to 160°C/lmm (discarded) and a main fraction 7.33g b.p. 160-215°C/lmm.
¹H NMR (CDCl₃) (ppm) 1.78 (m, 2H), 2.2 (br s, 2NH), 2.76 (m, 4H), 3.9 (s, 4H), 6.96 (m, 2H), 7.15 (d, 2H), 7.45 (m, 2H), 8.37 (d, 2H)
¹³C NMR (CDCl₃) 30.19, 47.77, 55.11, 121.59, 121.97, 136.12, 148.97, 159.81.

### 2,6-bis(pyridin-2-ylmethyl)-1,1,7,7-tetrakis(pyridin-2-yl)-2,6-diazaheptane (N,N'-Bis(Dipyrid-2-ylmethyl)-N,N'-bis(pyrid-2-ylmethyl)-1,3-diamino-propane)

A mixture of 1.7g dipyridyl-methylchloride (14), 1 g N,N'-bis(pyrid-2-ylmethyl)-1,3-diamino-propane (15) and 0.5g potassium carbonate in 20 ml acetonitrile was stirred and refluxed under argon for 48h. TLC (silica/ eluent CH2Cl2/MeOH(7N NH3) 90/10) indicated the reaction to be almost complete. The mixture was filtered and evaporated. The residue was chromatographed on silica using dichloromethane with increasing methanol concentration (up to 5%) to give 1.5g pure product as a yellow/brown glassy gum.
¹H NMR (CDCl₃) δ (ppm): 1.75 (m, 2H), 2.42 ppm (m, 4H), 3.73 (s, 4H), 5.16 (s, 2H), 7.02 (m, 6H), 7.4-7.6 (multiplets, 12H), 8.38 (m, 2H), 8.46 (m, 4H); ¹³C NMR (CDCl₃) (ppm): 23.5, 49.5, 57.1, 73.0, 121.6, 121.9, 122.7, 123.6, 136.1, 136.2, 148.8, 149.0, 160.4; ESP-Mass m/z: 615.3 (M+Na)⁺, 593.4 (M+H)⁺, 502.3 (M+H-CH2C5H4N), 425.2 (502.3+H-C5H4N).

### Fe-complex

To a solution of 0.11g of the ligand (see above) in 2ml MeOH and 2 ml acetonitrile was added 0.1g iron(II) perchlorate hexahydrate and 0.2g of sodium perchlorate. Ethyl acetate was allowed to diffuse into the mixture for over three days. Dark brown crystals were isolated from the mixture by filtration giving 20mg of the product after drying. UV/Vis (CH3CN) λₘₐₓ (ε a.u.): 695 nm (0.038), 493 nm (0.259), 471 nm (0.271), 334 nm (2.95). I.R. (Kbr, cm-1): 3421, 1607,1447, 1112, 1088, 792, 628.

### Compound 4: [Fe(L⁴)(CH₃CN)](ClO₄)₂

### 1,1-bis(pyridin-2-yl)-1-benzyl-N,N-bis(pyridin-2-ylmethyl)methylamine (L4) Compound 4 was synthesised as described elsewhere (EP 0909 809 A2)

### Compound 5

### 1,1-bis(pyridin-2yl)-N,N-bis(5-carboxymethyl-pyridin-2-ylmethyl)methylamine (L5)

Methyl 6-methylnicotinate (10 g. 66.2 mmol) was dissolved in dichloromethane (150 ml). 3-Chloroperoxybenzoic acid (17 g. 112 mmol) was added and the mixture was stirred for 3 hours at room temperature. Saturated NaHCO₃ solution (200 ml) was added and the mixture was stirred for an additional hour. The dichloromethane layer was separated and the aqueous layer was extracted with dichloromethane (2 × 100 ml). The combined dichloromethane layers were washed with saturated NaHCO₃ (aq) (100 ml), brine (100 ml) and dried (Na₂SO₄). After evaporation of the solvent methyl 6-(chloromethyl)nicotinate N-oxide (7.8 g, 51.0 mmol) was obtained as a creme colored solid, mp 90.4 - 90.8 °C, which was combined with p-toluenesulfonyl chloride (10.7 g, 56.1 mmol) and dioxane (100 ml) under an Argon atmosphere. The reaction mixture was heated under reflux for 1 night. After cooling to room temperature the solvent was evaporated and the residue dissolved in dichloromethane (200 ml). The solution was washed with saturated Na₂CO₃ (aq) (2 × 100 ml), brine (50 ml) and dried (Na₂SO₄). After evaporation of the solvent the product was purified by column chromatography (SiO2, using hexane/ethyl acetate 10:2.5 as an eluens) to give methyl 6-(chloromethyl)nicotinate (5.71 g, 46 % overall yield) as a slightly yellow solid. An analytically pure sample could be obtained by recrystallization from *n*-hexane, mp 63.5 - 63.8 °C; ¹H-NMR (CDCl₃) δ 3.94 (s. 3H), 4.70 (s, 2H), 7.58 (d, 1H, J = 8.4 Hz), 8.30 (dd, 1H, J = 8.1 Hz, J = 2.2 Hz), 9.08 (d, 1H, J = 1.5 Hz); Anal. Calcd. for C₈H₈ClNO₂: C 51.77, H 4.34, N 7.55; found: C 51.50, H 4.23, N 7.46.

A solution of di(2-pyridinyl)methylamine (555 mg, 3.0 mmol), methyl 6-(chloromethyl)nicotinate (1.7 g, 9.2 mmol) and *N*,*N*-diisopropylethylamine (1.6 ml, 9.2 mmol) was placed under argon and heated under reflux for 1 night. After evaporation of the solvent water (10 ml) was added and the product was extracted with ethyl acetate (3x 15 ml). The combined organic layers were washed with brine (10 ml), dried (Na₂SO₄) and the solvent removed *in vacuo.* Column chromotagraphy (Alox akt. I, ethyl acetate/hexane/triethylamine 10:5:1) afforded 1,1-bis(pyridin-2yl)-N,N-bis(5-carboxymethyl-pyridin-2-ylmethyl)methylamine (548 mg, 1.2 mmol, 40 %) as a slightly yellow oil. ¹H NMR (CDCl₃, 300 MHz) δ 3.90 (s, 6H), 4.04 (s, 4H), 5.32 (s, 1H), 7.13 (m, 2H), 7.60 (m, 2H), 8.16 (dd, 2H, J = 8.05 Hz, J = 2.2 Hz), 8.56 (d, 2 H, J = 4.8 Hz) 9.06 (d, 2H, J = 1.8 Hz); ¹³C NMR (CDCl₃, 50 MHz) δ 52.03 (q), 57.28 (t), 72.32 (d), 122.16 (d), 122.39 (d), 123.82 (d), 124.10 (s), 136.24 (d), 137.22 (d),149.25 (d), 150.15 (d), 159.48 (s), 164.33 (s), 165.69 (s); MS (CI): m/z 484 (M+1).

### [(L5)Fe(CH₃CN)](ClO₄)₂

To a solution of 1,1-bis(pyridin-2yl)-N,N-bis(5-carboxymethyl-pyridin-2-ylmethyl)methylamine (72 mg, 0.15 mmol) in acetonitrile (1.5 ml) was added a solution of Fe(ClO₄)₂·6H₂O (55 mg, 0.15 mmol) in methanol (1.5 ml). The solution was placed in an ethyl acetate bath and after 3 days [(L5)Fe(CH₃CN)](ClO₄)₂ (96 mg, 0.12 mmol, 82 %) was obtained as dark red crystals. ¹H NMR (CD₃CN, 300 MHz) δ 3.94 (s, 6H), 4.39 (d, 2H, J = 18.7 Hz), 4.51 (d, 2H, J = 19.0 Hz), 6.40 (s, 1H), 7.21 (d, 2H, J = 8.1 Hz), (t, 2H, J = 6.2 Hz), 7.91 (m, 4H), 8.14 (d, 2H, 8.1 Hz), 8.91 (d, 2H, J = 4.8 Hz), 9.48 (s, 2H); Anal. calcd for C₂₉H₂₈Cl₂Fe₁N₆O₁₂: C 44.69, H 3.62, N 10.78; found: C 44.28, H 3.69, N 10.63.

### Compound 6

### 1-[di(2-pyridin-2-yl)methyl]-4,7-dimethyl-1,4,7-triazacyclonane (L6)

To a solution of 80 mL n-BuLi in hexanes (2.5 M, 0.2 mol) was added 2-pyridylbromide (31.6 gram 0.2 mol) in 100 mL of ether at - 80°C - -60°C. The suspension was stirred for 1 h, and the temperature was allowed to rise to -45° C. Subsequently 2-pyridinecarboxaldehyde (21.42 gram 0.2 mol) in of ether (100 mL) was added during 30 min. To the thick slurry was added additional THF (200 mL) and the mixture was stirred for 1.5 h at -40° C - -30° C and then the mixture was allowed to warm up to -10 ° C. The mixture was poured into water (200 ml) and acidified with 2 M HCl to pH = 1-2 and the layers were separated. The aqueous layer was extracted twice with ether (100 mL) and neutralized with saturated Na₂CO_{3(aq)} to pH = 8. The aqueous layer was extracted CH₂Cl₂ (3x100 ml). Drying (Na₂SO₄) and evaporation of the solvent yielded a brown oil. Vacuum distillation (118 ° C, 0.2 mmHg) afforded di(2-pyidinyl)methanol (19.42 gram, 104.4 mmol, 52 %) as a yellow oil. ¹H-NMR (200 MHz, CDCl₃) δ 5.88 (s, 2H), 7.11 - 7.19 (m, 2H), 7.47 - 7.67 (m, 4H). 8.50 - 8.54 (m, 2H). ¹³C-NMR (50.3 MHz, CDCl₃) δ 75.0 (d), 121.0 (d), 122.5 (d), 136.8 (d), 1448.1 (d), 160.7 (s).

To di(2-pyridinyl)methanol (8.73 g, 46.94 mmol) in CH₃CN (100 ml) at 0 °C was added a solution of PPh₃ (14.77 gram, 56.32 mmol) in of CCl₄ (80 mL) in 1.5 h. The solution was left standing overnight. After addition of MeOH (10 ml) and stirring for 15 min the mixture was concentrated in vacuum to ca 50 ml. To the residue was added of water (100 ml) and the mixture was acidified with 2M HCl to pH = 1. and washed twice with 100 ml of CHCl₃ the aqueous layer was neutralized with K₂CO₃ and extracted 4 times with 75 ml of ether. Drying and evaporation of the solvent yielded 2-[chloro(2-pyidinyl)methyl]pyridine (5.41 gram, 56 %) as a pale brown solid. Analytically pure material was obtained by column chromatography on silica (ether). ¹H-NMR (200 MHz, CDCl₃) δ 6.20 (s,1H), 7.14 - 7.20 (m, 2H), 7.60 - 7.73 (m, 4H), 8.51 - 8.54 (m, 2H). ¹³C-NMR (50.3 MHz, CDCl₃) δ 62.84 (d), 121.3 (d), 121.5 (d), 135.7 (d), 147.7 (d), 156.9 (s). Anal. Cald. for C₁₁H₉ClN₂:C 64.56, H 4.43, Cl 17.32, N 13.69; Found: C 64.48, H 4.45, Cl 17.29, N 13.49.

A solution of bis(2-pyridyl)methyl chloride (170 mg, 0.83 mmol), 1,4-dimethyl-1,4,7-triazacyclonane (155 mg, 0.99 mmol) (ref. Koek et al, J.Chem.Soc., Dalton, Trans. 353 (1996)) and K₂CO₃ (136 mg, 0.99 mmol) in acetortitrile (10 ml) was placed under Ar and heated under reflux during 16 h. The reaction mixture was poored out in water (20 ml) and brought to pH>10 with NaOH. The aqueous solution was extracted with ethyl acetate (3 × 15 ml). The combined organic layers were dried (K₂CO₃) and the solvent was removed in vacuo to give 1-[di(2-pyridinyl)methyl]-4,7-dimethyl-1,4,7-triazonane (250 mg, 0.77 mmol, 93 %) as a slightly yellow oil. ¹H NMR (CDCl₃, 300 MHz) δ 2.28 (s, 6H), 2.60 (m, 4H), 2.79 (s, 4H), 2.81 (m, 4H), 5.07 (s, 1H), 7.06 (dt, 2H, J = 5.1 Hz, J = 3.3 Hz), 7.57 (m, 4H), 8.47 (d, 2H, J = 4.8 Hz); ¹³C NMR (CDCl₃, 50 MHz) δ 46.40 (q), 53.96 (t), 56.93 (t), 56.97 (t), 77.70 (d), 121.67 (d), 123.48 (d), 135.96 (d), 148.79 (d), 161.22 (s); HRMS calcd. for C₁₉H₂₇N₅ 325.227, found 325.227.

### [(L6)Fe(CH₃CN)](ClO₄)₂

To a solution of 1-[di(2-pyridinyl)methyl]-4,7-dimethyl-1,4,7-triazacyclonane (78 mg, 0.24 mmol) in acetonitrile (2 ml) was added a solution of Fe(ClO₄)₂·6H₂O (95 mg, 0.26 mmol) in methanol (2 ml). The solution was placed in an ethyl acetate bath and after 1 night [(L6)Fe(CH₃CN)](ClO₄)₂ (0.2 mmol, 85 %) was obtained as dark red crystals. ¹H NMR (CD₃CN, 300 MHz) δ 2.73 (s, 6H), 2.86 (m, 6H), 2.96 (m, 6H), 6.09 (s, 1H), 7.33 (m, 2H), 7.79 (d, 2H, J = 7.7 Hz), 7.88 (dt, 2H, J = 7.7 Hz, J = 1.1 Hz), 8.99 (d, 2H, J = 5.5 Hz); Anal. calcd for C₂₁H₃₀Cl₂FeN₆O₈: C 40.60, H 4.87, N 13.53; found: C 40.56, H 4.85, N 13.43.

### Compound 7

### 1-[1,1-di(2-pyridinyl)ethyl]-4,7-dimethyl-1,4,7-triazacyclonane (L7)

A solution of 1-[di(2-pyridinyl)methyl]-4,7-dimethyl-1,4,7-triazacyclononane (300 mg, 0.92 mmol) in ether/THF 1:1 (30 ml) was cooled to -80 °C and *t*-butyllithium (1.5 M in pentane. 0.65 ml, 0.97 mmol) was added. After for stirring for 20 min at -80 °C Mel (60 µL, 0.96 mmol) and the solution was allowed to warm up to room temperature overnight. After removal of the solvent CHCl₃ (30 ml) was added and the solution was washed with saturated NaHCO₃ (aq) (20 ml) and brine (20 ml), and dried (Na₂SO₄). Evaporation of the solvent afforded 1-[1,1-di(2-pyridinyl)ethyl]-4,7-dimethyl-1,4,7-triazacyclonane (300 mg, 0.88 mmol, 96 %) as a slightly orange solid, which was used without further purification. ¹³C NMR (CDCl₃, 50 MHz) δ 14.1 (q), 45.4 (q), 50.0 (t), 55.3 (t), 56.6 (t), 60.3 (s), 122.6 (d), 123.8 (d), 136.8 (d), 148.8 (d), 162.6 (s); MS(EI): 339 (M⁺).

### [(L7)Fe(CH₃CN)](ClO₄)₂

To a solution of 1-[1,1-di(2-pyridin-2-yl)ethyl]-4,7-dimethyl-1,4,7-triazacyclonane (112 mg, 0.33 mmol) in acetonitrile (6 ml) was added Fe(ClO₄)₂·6H₂O (143 mg, 0.39 mmol). The solution was placed in an ethyl acetate bath and after 1 night [(L7)Fe(CH₃CN)](ClO₄)₂ (90 mg, 0.14 mmol, 43 %) was obtained as red microcrystals. ¹H NMR (CD₃CN, 300 MHz) δ 2.24 (s, 3H), 2.67 (m, 6H), 2.70 (s, 6H), 2.97 (m, 6H), 7.33 (m, 2H), 7.63 (m, 2H), 7.90 (m, 2H), 9.01 (d, 2H, J = 5.5 Hz).

### Compound 8

### 2,2,4,4-tetrakis(pyridin-2-yl)-3-azapentane (L8)

Under vigorous stirring and N₂-atmosphere, 1 mL of a 3 M solution of MeMgBr in Et₂O was added dropwise to a solution of 300 mg (0.749 mmol) of 1,3,3-tris(2-pyridyl)-3*H*-imidazo[1,5-*a*]pyridin-4-ium (TPIP) in 20 mL of dry toluene (ref TPIP: M.Renz, et al., *J Chem. Soc., Chem. Commun.* 1998, 1635.). After 2 h, 2 mL of a saturated NH₄Cl solution was added and the solvent evaporated. The residue was dissolved in 10 mL CH₂Cl₂ and washed with 10 mL of a 2 N NaOH solution. After drying with MgSO₄, the solvent was evaporated, the residue dissolved in 1 mL CH₂Cl₂ and exposed to a pentane atmosphere overnight. 209 mg (73%) of L⁸ were obtained as colorless crystals. ¹H NMR (300 MHz, CDCl₃, 25 °C); d = 1.48 (s, 6 H), 5.96 (brs, 1 H, N-H), 7.08 (ddd, J = 1.7, 4.8, 6.6 Hz, 4 H, 2-H), 7.56 (dt, *J* = 1.8, 8.1 Hz, 4 H, 3-H), 7.61 (ddd, *J* = 1.1, 1.7, 8.1 Hz, 4 H, 4-H). 8.55 (ddd, *J* = 1.1, 1.7, 4.8 Hz, 4 H, 1-H). - ¹³C NMR (75 MHz, CDCl₃, 25°C); d = 26.3 (q, C-7), 65.6 (s, C-6), 121,5 (d, C-2), 122,3 (d, C-4), 136.4 (d, C-3), 148.5 (d, C-1), 168.7 (s, C-5). - FAB-MS, *m*/*z* (%): 382 (57) [M+1], 303 (16) [M-py], 183 [dipyridylethyl].

### Compound 11

### 1.1 bis-[pyridyl-2yl]-N,N-bis[benzimidazol-2yl-methylenyl]-methyl amine (L9)

### Preparation Dipyridyl-methylamine

Dipyridyl ketone (25.5g, 0.138mol, from Aldrich) and hydroxylamine hydrochloride (20g) were added to pyridine (120ml). The mixture was stirred and refluxed for 4h. allowed to cool to 20°C and concentrated by evaporation in vacuo. The residue was poored into 1 l of ice water and after stirring a precipitate formed. After 15min the precipitate was isolated by filtration and dried in vacuo at 60°C (drying is not strictly necessary as it can be used wet in the next step). This product was used without further purification in the next step.
In a 2l flask the product was dissolved in ethanol (250ml) and concentrated ammonia (400ml), water (250ml) and ammonium acetate (10 g) were added. The mixture was heated to 90°C while stirring with a mechanical stirrer. Zinc powder (37.5g) was added in small portions to the stirred mixture over a 1h period. After the addition was complete stirring was contnued for 3h. TLC (silica, eluent ammonia/butanol 70:30) showed conversion to be complete and the mix was allowed to cool to 20°C. filtered over celite and concentrated. Sodium hydroxide solution (20%. 100ml) was added to the concentrate and the mixture was extracted three times with ether.
(The aqueous layer should be strongly alkaline, in some cases more extractions were needed to obtain a good yield. The use of CH₂Cl₂ instead of ether is preferred as extraction is more efficient)
The ether lavers were combined and washed with saturated sodium chloride solution, dried over sodium sulfate, filtered and evaporated to give 21g (81.9% if pure) of a light yellow oil
¹H NMR (CDCl₃) (ppm): 2.50 (bs, 2H, NH2); 5.30 (s, 1H, Py2C-H); 7.05 (m, 2H, Py-H); 7.37 (m, 2H, Py-H); 7.58 (m, 2H, Py-H); 8.51 (m, 2H, Py-H).
¹³C NMR (CDCl₃) (ppm): 62.6, 122.0, 122.3, 136.9, 149.4 and 163.0

### N2Py-diacetate

To a cooled solution of sodium hydroxide (3.5 g in 3 ml water) was added 4.2 chloroacetic acid. Subsequently 3.7 g (20 mmol) of dipyrridylmethane in 6 ml water was added. The reaction was stirred and monitored by TLC (30% ammonia / 70% MeOH). After 5 days still starting product was observed and again chloroacetic acid prenuetralized in alkaline was added in a couple of portions over time till TLC indicate that all starting material was converted. After workup a mixture of product, triethylamine (needed to extract the produuct into an organic phase) and glycolate was obtained. The product was used without further purification.

### 1,1 bis-[pyridyl-2yl]-N,N'-bis-[benzimidazol-2yl-methylenyl]-methyl amine

To 2.5 g of the mixture obtained as described above, was added 1.4 g o-phenylene diamine and placed in a 195C oil bath. After 25 minutes the mixture was allowed to cool, taken up in dichloromethane and washed with ammonia. The dichloromethane layer was evaporated giving a dark red oil. Chromatography (SiO2, with CH2Cl2/MeOH gradient) gave 0.56 g product.
¹H NMR (CDCl₃) (ppm): 4.0 (s, 4H, CH2); 5.30 (s, 1H, Py2C-H); 7.06 (m, 2H, Py-H); 7.21 (m, 4H, Ar-CH), 7.39 (m, 2H, Py-H); 7.50 (m, 2H, Py-H); 7.60 (m, 4H, Ar-CH), 8.48 (m, 2H, Py-H).
¹³C NMR (CDCl₃) (ppm): 49.3, 72.5, 115.2, 115.6, 122.6, 122.7, 123.0, 124.5, 137.3, 137.9, 138.3, 141.1, 149.1 152.2 and 158.7

### Compounds 16 and 17

### 2,6-bis(methoxy-bis(pyridin-2-yl)methyl)pyridin (L10) and 2,6-bis(hydroxy-bis-pyridin-2-yl)-methyl)pyridin (L11) were synthesised as published elsewhere (M.E. de Vries, B.L. Feringa, et al., Chem Comm, 1549 (1997).

### Experimental:

In an aqueous solution containing 10 mM carbonate buffer (pH 10) without and with 0.6 g/l Na-LAS (linear alkylbenzene sulfonate) or containing 10 mM borate buffer (pH 8) without and with 0.6 g/l NaLAS, tomato-soya oil stained cloths (6x6 cm) were added and stirred for 30 minutes at 30 °C (blanks). In a second series of experiments, the same tests were done in the presence of 10 µM of compound **1-7** or 20 µM of ligand L9, L10, or L11 in combination with Mn, Fe, Co or Cu perchlorate salt

The cloths were measured immediately after the wash (Table 1) or after 24 h storage in a dark room under ambient conditions (Table 2).

After the wash, the cloths were rinsed with water and subsequently dried at 30 °C and the change in colour was measured immediately after drying with a Linotype-Hell scanner (ex Linotype). The change in colour (including bleaching) is expressed as the ΔE value. The measured colour difference (ΔE) between the washed cloth and the unwashed cloth is defined as follows:

ΔE = [(ΔL)² +(Δa)² +(Δb)²]^{1/2}

wherein ΔL is a measure for the difference in darkness between the washed and unwashed test cloth; Δa and Δb are measures for the difference in redness and yellowness respectively between both cloths. With regard to this colour measurement technique, reference is made to Commission International de l'Eclairage (CIE): Recommendation on Uniform Colour Spaces, colour difference equations, psychometric colour terms, supplement no 2 to CIE Publication, no 15, Colormetry. Bureau Central de la CIE. Paris 1978.

The results are shown in Tables 1 and 2 below:

**Table 1**

| Bleach values expressed as ΔE obtained for the tomato stains for the various compounds | | | | |
|---|---|---|---|---|
| | pH 8 - LAS | pH 8 + LAS | pH 10-LAS | pH 10 + LAS |
| Blank | 1 | 2 | 1 | 3 |
| Compound **1** | 1 | 12 | 1 | 4 |
| Compound **2** | 6 | 15 | 2 | 6 |
| Compound **3** | 2 | 12 | 2 | 5 |
| Compound **4** | 16 | 16 | 16 | 16 |
| Compound **5** | 18 | 16 | 6 | 11 |
| Compound **6** | 1 | 6 | 1 | 7 |
| Compound **7** | 2 | 10 | 2 | 11 |
| Compound **8** | 3 | 16 | 5 | 19 |
| Compound **9** | 3 | 13 | 5 | 15 |
| Compound **10** | 3 | 9 | 3 | 4 |
| Compound **11** | 13 | 12 | 13 | 18 |
| Compound **12** | 17 | 14 | 8 | 17 |
| Compound **13** | 14 | 11 | 6 | 5 |
| Compound **14** | 11 | 6 | 4 | 8 |
| Compound **15** | 13 | 8 | 8 | 13 |

**Table 2**

| Bleach values expressed as ΔE obtained for the tomato stains after storage for 24 h in the dark | | | | |
|---|---|---|---|---|
| | pH 8 - LAS | pH 8 + LAS | pH 10 - LAS | pH 10 + LAS |
| Blank | 2 | 4 | 2 | 4 |
| Compound **16** | 2 | 15 | 3 | 15 |
| Compound **17** | 5 | 12 | 8 | 13 |

## Claims

1. A bleaching composition comprising, in an aqueous medium, atmospheric oxygen and a ligand which forms a complex with a transition metal, the complex catalysing bleaching of a substrate by the atmospheric oxygen, wherein the aqueous medium is substantially devoid of peroxygen bleach or a peroxy-based or -generating bleach system,
wherein the ligand forms a complex of the general formula (A1):
[MₐLₖXₙ]Yₘ (A1)
in which:
M represents a metal selected from Mn(II)-(III)-(IV)-(V), Cu(I)-(II)-(III), Fe(II)-(III)-(IV)-(V), Co(I)-(II)-(III), Ti(II)-(III)-(IV), V(II)-(III)-(IV)-(V), Mo(II)-(III)-(IV)-(V)-(VI) and W(IV)-(V)-(VI);
X represents a coordinating species selected from any mono, bi or tri charged anions and any neutral molecules able to coordinate the metal in a mono, bi or tridentate manner;
Y represents any non-coordinated counter ion:
a represents an integer from 1 to 10;
k represents an integer from 1 to 10;
n represents an integer from 1 to 10;
m represents zero or an integer from 1 to 20; and
L represents a ligand of the general formula (I), or its protonated or deprotonated analogue: 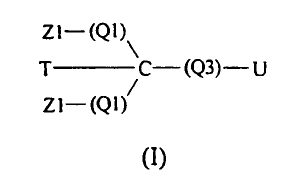
wherein
Z1 groups independently represent a coordinating group selected from hydroxy, amino, -NHR or -N(R)₂ (wherein R=C₁₋₆-alkyl), carboxylate, amido, -NH-C(NH)NH₂, hydroxyphenyl, a heterocyclic ring optionally substituted by one or more functional groups E or a heteroaromatic ring optionally substituted by one or more functional groups E, the heteroaromatic ring being selected from pyridine, pyrimidine, pyrazine, pyrazole, imidazole, benzimidazole, quinoline, quinoxaline, triazole, isoquinoline, carbazole, indole, isoindole, oxazole and thiazole;
Q1 and Q3 independently represent a group of the formula: 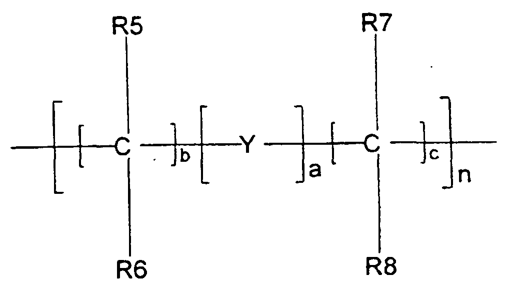 wherein
5 ≥ a+b+c ≥ 1; a=0-5; b=0-5; c=0-5; n=0 or 1 (preferably n=0);
Y independently represents a group selected from -O-, -S-, -SO-, -SO₂-, -C(O)-, arylene, alkylene, heteroarylene, heterocycloalkylene, -(G)P-, -P(O)- and -(G)N-, wherein G is selected from hydrogen, alkyl, aryl, arylalkyl, cycloalkyl, each except hydrogen being optionally substituted by one or more functional groups E;
R5, R6, R7, R8 independently represent a group selected from hydrogen, hydroxyl, halogen, -R and -OR, wherein R represents alkyl, alkenyl, cycloalkyl, heterocycloalkyl, aryl, heteroaryl or a carbonyl derivative group, R being optionally substituted by one or more functional groups E,
or R5 together with R6, or R7 together with R8, or both, represent oxygen,
or R5 together with R7 and/or independently R6 together with R8, or R5 together with R8 and/or independently R6 together with R7, represent C₁₋₆-alkylene optionally substituted by C₁₋₄-alkyl. -F, -Cl, -Br or -I;
E independently represents a functional group selected from -F, -Cl, -Br, -I, -OH, -OR', -NH₂, -NHR', -N(R')₂, -N(R')₃⁺, -C(O)R', -OC(O)R', -COOH, -COO⁻(Na⁺, K⁺), -COOR', -C(O)NH₂, -C(O)NHR', -C(O)N(R')₂, heteroaryl, -R', -SR', -SH, -P(R')_{2,} -P(O)(R')₂, -P(O)(OH)₂, -P(O)(OR')₂, -NO₂, -SO₃H, -SO₃⁻(Na⁺, K⁺), -S(O)₂R', -NHC(O)R', and -N(R')C(O)R', wherein R' represents cycloalkyl, aryl, arylalkyl, or alkyl optionally substituted by -F, -Cl, -Br, -I, -NH₃⁺, -SO₃H, -SO₃⁻(Na⁺, K⁺), -COOH, -COO⁻ (Na⁻, K⁺), -P(O)(OH)₂, or -P(O)(O⁻(Na⁺, K⁺))₂;
T represents a non-coordinated group selected from hydrogen, hydroxyl, halogen, -R and -OR, wherein R represents alkyl, alkenyl, cycloalkyl, heterocycloalkyl, aryl, heteroaryl or a carbonyl derivative group, R being optionally substituted by one or more functional groups E:
U represents either a non-coordinated group T independently defined as above or a coordinating group of the general formula (II), (III) or (IV): 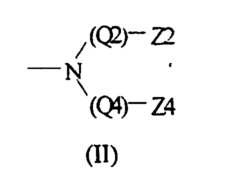 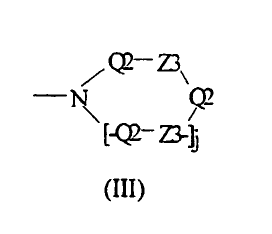 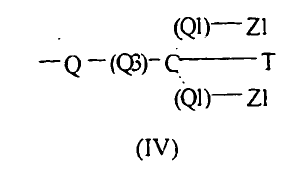 wherein
Q2 and Q4 are independently defined as for Q1 and Q3;
Q represents -N(T)- (wherein T is independently defined as above), or an optionally substituted heterocyclic ring or an optionally substituted heteroaromatic ring selected from pyridine, pyrimidine, pyrazine, pyrazole, imidazole, benzimidazole, quinoline, quinoxaline, triazole, isoquinoline, carbazole, indole, isoindole, oxazole and thiazole:
Z2 is independently defined as for Z1;
Z3 groups independently represent -N(T)- (wherein T is independently defined as above);
Z4 represents a coordinating or non-coordinating group selected from hydrogen, hydroxyl, halogen, -NH-C(NH)NH₂, -R and -OR, wherein R= alkyl, alkenyl, cycloalkyl, heterocycloalkyl, aryl, heteroaryl or a carbonyl derivative group, R being optionally substituted by one or more functional groups E, or Z4 represents a group of the general formula (IIa): 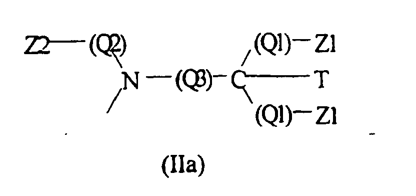 and
1≤j<4.

2. A bleaching composition according to claim 1, wherein the medium has a pH value in the range from pH 6 to 11, preferably in the range from pH 8 to 10.

3. A bleaching composition according to claim 1 or claim 2, wherein the medium is substantially devoid of a transition metal sequestrant.

4. A bleaching composition according to any of claims 1 to 3, wherein the medium further comprises a surfactant.

5. A bleaching composition according to any of claims 1 to 4, wherein the medium further comprises a builder.

6. A bleaching composition according to any of claims 1 to 5, wherein the composition comprises a preformed complex of the ligand and a transition metal.

7. A bleaching composition according to any of claims 1 to 5, wherein the ligand is present as a free ligand that complexes with a transition metal present in the water.

8. A bleaching composition according to any of claims 1 to 5, wherein the ligand is present as a free ligand that complexes with a transition metal present in the substrate.

9. A bleaching composition according to any of claims 1 to 5, wherein the composition comprises the ligand present as a free ligand or a transition metal-substitutable metal-ligand complex, and a source of transition metal.

10. A bleaching composition according to any preceding claim, wherein Z1, Z2 and Z4 independently represent an optionally substituted heterocyclic ring or an optionally substituted heteroaromatic ring selected from pyridine, pyrimidine, pyrazine, pyrazole, imidazole, benzimidazole, quinoline, quinoxaline, triazole, isoquinoline, carbazole, indole, isoindole, oxazole and thiazole.

11. A bleaching composition according to any preceding claim, wherein the Z1, Z2 and Z4 independently represent groups selected from optionally substituted pyridin-2-yl, optionally substituted imidazol-2-yl, optionally substituted imidazol-4-yl, optionally substituted pyrazol-1-yl, and optionally substituted quinolin-2-yl.

12. A bleaching composition according to any preceding claim, wherein Z1, Z2 and Z4 each represent optionally substituted pyridin-2-yl.

13. A bleaching composition according to any preceding claim, wherein Z1, Z2 and Z4 are optionally substituted by a group selected from C₁₋₄-alkyl, aryl, arylalkyl, heteroaryl, methoxy, hydroxy, nitro, amino, carboxyl, halo, and carbonyl.

14. A bleaching composition according to any preceding claim, wherein one or more of Z1, Z2 and Z4 are substituted by a methyl group.

15. A bleaching composition according to any preceding claim, wherein the Z1 groups represent identical groups.

16. A bleaching composition according to any preceding claim, wherein R5, R6, R7, R8 independently represent a group selected from -H, hydroxy-C₀-C₂₀-alkyl, halo-Co-C₂₀-alkyl, nitroso, formyl-C₀-C₂₀-alkyl, carboxyl-C₀-C₂₀-alkyl and esters and salts thereof, carbamoyl-C₀-C₂₀-alkyl, sulfo-C₀-C₂₀-alkyl and esters and salts thereof, sulfamoyl-C₀-C₂₀-alkyl, amino-C₀-C₂₀-alkyl, aryl-C₀-C₂₀-alkyl, C₀-C₂₀-alkyl, alkoxy-C₀-C₈-alkyl, carbonyl-C₀-C₆-alkoxy, and C₀-C₂₀-alkylamide.

17. A bleaching composition according to any preceding claim, wherein each Q1 represents a covalent bond or C1-C4-alkylene, preferably a covalent bond, methylene or ethylene, more preferably a covalent bond.

18. A bleaching composition according to any preceding claim, wherein each Q3 represents a covalent bond or C1-C4-alkylene, preferably a covalent bond.

19. A bleaching composition according to any preceding claim, wherein T represents hydrogen, hydroxy, methyl, ethyl, benzyl, or methoxy.

20. A bleaching composition according to any of claims 1 to 19, wherein U represents a coordinating group of the general formula (II):

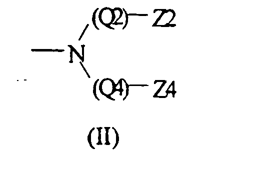

21. A bleaching composition according to claim 20, wherein Z2 represents an optionally substituted heterocyclic ring or an optionally substituted heteroaromatic ring selected from pyridine, pyrimidine, pyrazine, pyrazole, imidazole, benzimidazole, quinoline, quinoxaline, triazole, isoquinoline, carbazole, indole, isoindole, oxazole and thiazole, preferably optionally substituted pyridin-2-yl or optionally substituted benzimidazol-2-yl, and wherein Z4 represents an optionally substituted heterocyclic ring or an optionally substituted heteroaromatic ring selected from pyridine, pyrimidine, pyrazine, pyrazole, imidazole, benzimidazole, quinoline, quinoxaline, triazole, isoquinoline, carbazole, indole, isoindole, oxazole and thiazole, preferably optionally substituted pyridin-2-yl, or an non-coordinating group selected from hydrogen, hydroxy, alkoxy, alkyl, alkenyl, cycloalkyl, aryl, or benzyl.

22. A bleaching composition according to claim 21, wherein L represents a ligand selected from:
1,1-bis(pyridin-2-yl)-N-methyl-N-(pyridin-2-ylmethyl)methylamine;
1,1-bis(pyridin-2-yl)-N,N-bis(6-methyl-pyridin-2-ylmethyl)methylamine;
1,1-bis(pyridin-2-yl)-N,N-bis(5-carboxymethyl-pyridin-2-ylmethyl)methylamine;
1,1-bis(pyridin-2-yl)-1-benzyl-N,N-bis(pyridin-2-ylmethyl)methylamine; and 1,1-bis(pyridin-2yl)-N,N-bis(benzimidazol-2-ylmethyl)methylamine.

23. A bleaching composition according to claim 20, wherein Z4 represents a group of the general formula (IIa): 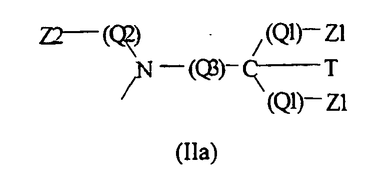 and Q4 represents optionally substituted alkylene, preferably -CH₂-CHOH-CH₂- or -CH₂-CH₂-CH₂-.

24. A bleaching composition according to claim 23, wherein L represents the ligand: 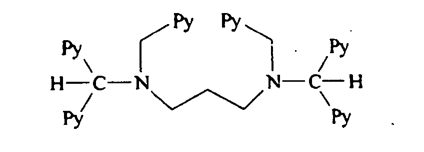 wherein -Py represents pyridin-2-yl.

25. A bleaching composition according to any of claims 1 to 19, wherein U represents a coordinating group of the general formula (III): 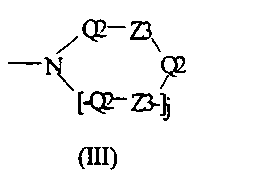 wherein j is 1 or 2, preferably 1.

26. A bleaching composition according to claim 25, wherein each Q2 represents -(CH₂)ₙ- (n=2-4), and each Z3 represents -N(R)- wherein R = -H or C₁₋₄-alkyl.

27. A bleaching composition according to claim 26, wherein L represents a ligand selected from: 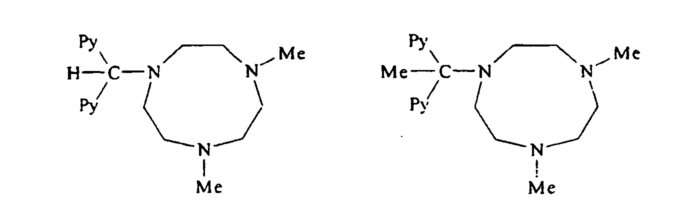 wherein -Py represents pyridin-2-yl.

28. A bleaching composition according to any of claims 1 to 19, wherein U represents a coordinating group of the general formula (IV):

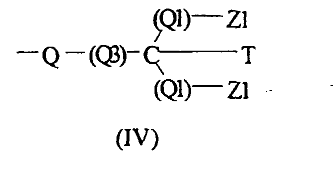

29. A bleaching composition according to claim 28, wherein Q represents -N(T)-(wherein T= -H, methyl, or benzyl) or pyridin-diyl.

30. A bleaching composition according to claim 29, wherein L represents a ligand selected from: 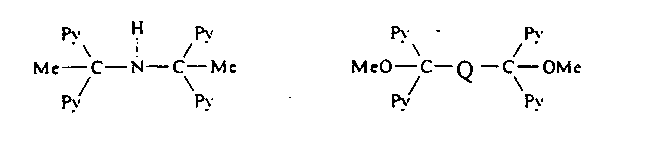 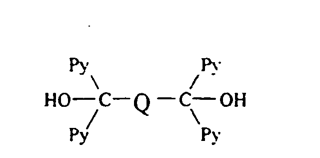 wherein -Py represents pyridin-2-yl, and -Q- represents pyridin-2,6-diyl.

31. A bleaching composition according to any preceding claim, wherein the composition comprises a mixture of the ligand L and a metal salt MXₙ in which n=1-5, preferably 1-3.

32. A ligand L as defined in any of the preceding claims with the proviso that U represents a non-coordinated group T or a coordinated group of the formula (II) or (III), and if U represents a coordinating group of formula (II) and Z1=Z2=Z4=unsubstituted pyridin-2-yl, T is not hydrogen, methyl or benzyl.

33. A ligand selected from:
1,1-bis(pyridin-2-yl)-N-methyl-N(pyridin-2-ylmethyl)methylamine;
1,1-bis(pyridin-2-yl)-N,N-bis(6-methyl-pyridin-2-ylmethyl)methylamine;
1,1-bis(pyridin-2-yl)-N,N-bis(5-carboxymethyl-pyridin-2-yl)methylamine;
1,1-bis(pyridin-2yl)-N,N-bis(benzimidazol-2-ylmethyl)methylamine;
2,6-bis(pyridin-2ylmethyl)-1,1,7,7-tetrakis(pyridin-2-yl)-2,6-diazaheptane; or a ligand selected from: 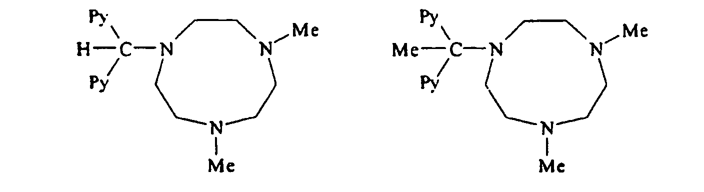 wherein -Py represents pyridin-2-yl.

34. A transition-metal complex of the general formula (A1):
[MₐLₖXₙ]Yₘ (A1)
in which:
M represents a metal selected from Mn(II)-(III)-(IV)-(V), Cu(I)-(II)-(III), Fe(II)-(III)-(IV)-(V), Co(I)-(II)-(III), Ti(II)-(III)-(IV), V(II)-(III)-(IV)-(V), Mo(II)-(III)-(IV)-(V)-(VI) and W(IV)-(V)-(VI);
X represents a coordinating species selected from any mono, bi or tri charged anions and any neutral molecules able to coordinate the metal in a mono, bi or tridentate manner;
Y represents any non-coordinated counter ion;
a represents an integer from 1 to 10;
k represents an integer from 1 to 10;
n represents an integer from 1 to 10;
m represents zero or an integer from 1 to 20; and
L represents a ligand as defined in claim 32 or 33, or its protonated or deprotonated analogue.

35. A method of bleaching a substrate comprising applying to the substrate, in an aqueous medium, a ligand which forms a complex with a transition metal, the complex catalysing bleaching of the substrate by atmospheric oxygen, wherein the ligand is as defined in any of claims 1 to 33.

36. A method according to claim 35, wherein the majority of the bleaching species in the medium (on an equivalent weight basis) is derived from the atmospheric oxygen.

37. A method according to claim 35 or claim 36, wherein the medium is substantially devoid of peroxygen bleach or a peroxy-based or -generating bleach system.

38. A method according to any of claims 35 to 37, wherein the aqueous medium is agitated.

39. A method according to any of claims 35 to 38, wherein the medium is as defined in any of claims 2 to 5.

40. Use of a ligand which forms a complex with a transition metal as a catalytic bleaching agent for a substrate in an aqueous medium substantially devoid of peroxygen bleach or a peroxy-based or -generating bleach system, the complex catalysing bleaching of the substrate by the atmospheric oxygen wherein the ligand is as defined in any of claims 1 to 33.

41. A dry textile having a ligand as defined in any of claims 1 to 33 applied or deposited thereon, whereby bleaching by atmospheric oxygen is catalysed on the textile.

## Patentansprüche

1. Bleichzusammensetzung, umfassend in einem wässrigen Medium atmosphärischen Sauerstoff und einen Liganden, der einen Komplex mit einem Übergangsmetall bildet, wobei der Komplex das Bleichen eines Substrats durch den atmosphärischen Sauerstoff katalysiert, worin das wässrige Medium im Wesentlichen frei von Persauerstoffbleichmittel oder einem auf Peroxy basierenden oder erzeugenden Bleichmittelsystem ist,
worin der Ligand einen Komplex mit der allgemeinen Formel (A1) bildet:
[MₐLₖXₙ]Yₘ (A1)
worin:
M ein Metall, ausgewählt aus Mn(II)-(III)-(IV)-(V), Cu(I)-(II)-(III), Fe(II)-(III)-(IV)-(V), Co(I)-(II)-(III), Ti(II)-(III)-(IV), V(II)-(III)-(IV)-(V), Mo(II)-(III)-(IV)-(V)-(VI) und W(IV)-(V)-(VI), wiedergibt;
X eine koordinierende Spezies, ausgewählt aus beliebigen ein-, zwei- oder dreifach geladenen Anionen und beliebigen neutralen Molekülen, die das Metall in einer ein-, zwei- oder dreizähnigen Weise koordinieren können, wiedergibt;
Y beliebiges nicht-koordinierendes Gegenion wiedergibt;
a eine ganze Zahl von 1 bis 10 wiedergibt;
k eine ganze Zahl von 1 bis 10 wiedergibt;
n eine ganze Zahl von 1 bis 10 wiedergibt;
m null oder eine ganze Zahl von 1 bis 20 wiedergibt;
und
L einen Liganden der allgemeinen Formel (I) wiedergibt; oder dessen protoniertes oder deprotoniertes Analoges: 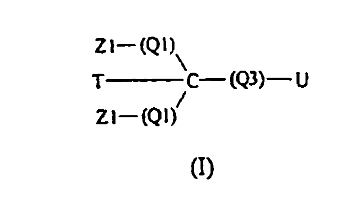
worin
Gruppen Z1 unabhängig eine koordinierende Gruppe, ausgewählt aus Hydroxy, Amino, -NHR oder -N(R)₂ (worin R=C₁₋₆-Alkyl), Carboxylat, Amido, -NH-C(NH)NH₂, Hydroxyphenyl, einem heterocyclischen Ring, gegebenenfalls substituiert mit einer oder mehreren funktionellen Gruppen E, oder einem heteroaromatischen Ring, gegebenenfalls substituiert mit einer oder mehreren funktionellen Gruppen E, wiedergibt, wobei der heteroaromatische Ring aus Pyridin, Pyrimidin, Pyrazin, Pyrazol, Imidazol, Benzimidazol, Chinolin, Chinoxalin, Triazol, Isochinolin, Carbazol, Indol, Isoindol, Oxazol und Thiazol ausgewählt ist;
Q1 und Q3 unabhängig eine Gruppe der Formel: 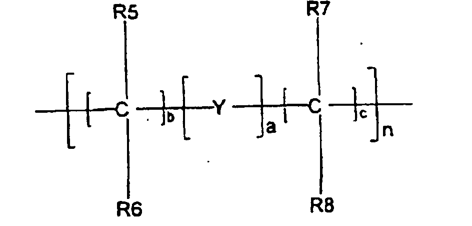 wiedergeben,
worin
5 ≥ a+b+c ≥ 1; a=0-5; b=0-5; c=0-5; n=0 oder 1 (vorzugsweise n=0);
Y unabhängig eine Gruppe, ausgewählt aus -O-, -S-, -SO-, -SO₂-, -C(O)-, Arylen, Alkylen, Heteroarylen, Heterocycloalkylen, -(G)P-, -P(O)- und -(G)N-, wiedergibt, worin G ausgewählt ist aus Wasserstoff, Alkyl, Aryl, Arylalkyl, Cycloalkyl, jeweils, ausgenommen Wasserstoff, gegebenenfalls mit einer oder mehreren funktionellen Gruppen E substituiert;
R5, R6, R7, R8 unabhängig eine Gruppe, ausgewählt aus Wasserstoff, Hydroxyl, Halogen, -R und -OR, wiedergeben, worin R Alkyl, Alkenyl, Cycloalkyl, Heterocycloalkyl, Aryl, Heteroaryl oder eine Gruppe von einem Carbonylderivat wiedergibt, wobei R gegebenenfalls mit einer oder mehreren funktionellen Gruppen E substituiert ist;
oder R5 zusammen mit R6 oder R7 zusammen mit R8 oder beide Sauerstoff wiedergeben,
oder R5 zusammen mit R7 und/oder unabhängig R6 zusammen mit R8 oder R5 zusammen mit R8 und/oder unabhängig R6 zusammen mit R7 C₁₋₆-Alkylen, gegebenenfalls substituiert mit C₁₋₄-Alkyl, -F, -Cl, -Br oder -I, wiedergeben;
E unabhängig eine funktionelle Gruppe, ausgewählt aus -F, -Cl, -Br, -I, -OH, -OR', -NH₂, -NHR', -N(R')₂, -N(R')₃⁺, -C(O)R', -OC(O)R', -COOH, -COO⁻(Na⁺,K⁺), -COOR' , -C(O)NH₂, -C(O)NHR', -C(O)N(R')₂, Heteroaryl, -R', -SR', -SH, -P(R')₂, -P(O)(R')₂, -P(O)(OH)₂, -P(O)(OR')₂, -NO₂, -SO₃H, -SO₃⁻(Na⁺,K⁺), -S(O)₂R', -NHC(O)R' und -N(R')C(O)R', wiedergibt, worin R' Cycloalkyl, Aryl, Arylalkyl oder Alkyl, gegebenenfalls substituiert mit -F, -Cl, -Br, -I, -NH₃⁺, -SO₃H, -SO₃⁻(Na⁺,K⁺), -COOH, -COO⁻(Na⁺,K⁺), -P(O)(OH)₂ oder -P(O)(O⁻(Na⁺,K⁺))₂, wiedergibt;
T eine nicht-koordinierte Gruppe, ausgewählt aus Wasserstoff, Hydroxyl, Halogen, -R und -OR, wiedergibt, worin R Alkyl, Alkenyl, Cycloalkyl, Heterocycloalkyl, Aryl, Heteroaryl oder eine Carbonyl-abgeleitete Gruppe wiedergibt, wobei R gegebenenfalls mit einer oder mehreren funktionellen Gruppen E substituiert ist;
U entweder eine nicht-koordinierte Gruppe T, unabhängig definiert, wie vorstehend oder eine koordinierende Gruppe der allgemeinen Formel (II), (III) oder (IV) wiedergibt; 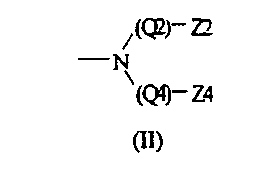 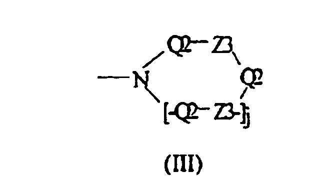 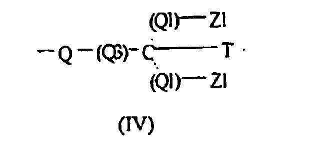 worin
Q2 und Q4 unabhängig wie für Q1 und Q3 definiert sind;
Q -N(T)- (worin T unabhängig wie vorstehend definiert ist) oder einen gegebenenfalls substituierten heterocyclischen Ring oder einen gegebenenfalls substituierten heteroaromatischen Ring, ausgewählt aus Pyridin, Pyrimidin, Pyrazin, Pyrazol, Imidazol, Benzimidazol, Chinolin, Chinoxalin, Triazol, Isochinolin, Carbazol, Indol, Isoindol, Oxazol und Thiazol, wiedergibt;
Z2 unabhängig wie für Z1 definiert ist;
Gruppen Z3 unabhängig -N(T)- (worin T unabhängig wie vorstehend definiert ist) wiedergeben;
Z4 eine koordinierende oder nicht-koordinierende Gruppe, ausgewählt aus Wasserstoff, Hydroxyl, Halogen, -NH-C(NH)NH₂, -R und -OR, wiedergibt, worin R= Alkyl, Alkenyl, Cycloalkyl, Heterocycloalkyl, Aryl, Heteroaryl oder eine Carbonyl-abgeleitete Gruppe, wobei R gegebenenfalls mit einer oder mehreren funktionellen Gruppen E substituiert ist, oder Z4 eine Gruppe der allgemeinen Formel (IIa) 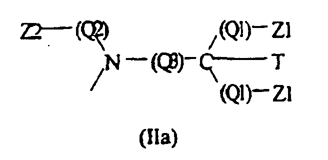 wiedergibt;
und
1 ≤ j < 4.

2. Bleichzusammensetzung nach Anspruch 1, worin das Medium einen pH-Wert im Bereich von pH 6 bis 11, vorzugsweise im Bereich von pH 8 bis 10, aufweist.

3. Bleichzusammensetzung nach Anspruch 1 oder Anspruch 2, worin das Medium im Wesentlichen frei von einem Übergangsmetallmaskierungsmittel ist.

4. Bleichzusammensetzung nach einem der Ansprüche 1 bis 3, worin das Medium weiterhin ein Tensid umfasst.

5. Bleichzusammensetzung nach einem der Ansprüche 1 bis 4, worin das Medium weiterhin einen Builder umfasst.

6. Bleichzusammensetzung nach einem der Ansprüche 1 bis 5, worin die Zusammensetzung einen vorgebildeten Komplex von dem Liganden und einem Übergangsmetall umfasst.

7. Bleichzusammensetzung nach einem der Ansprüche 1 bis 5, worin der Ligand als ein freier Ligand vorliegt, der mit einem in dem Wasser vorliegenden Übergangsmetall komplexiert.

8. Bleichzusammensetzung nach einem der Ansprüche 1 bis 5, worin der Ligand als ein freier Ligand vorliegt, der mit einem in dem Substrat vorliegenden Übergangsmetall komplexiert.

9. Bleichzusammensetzung nach einem der Ansprüche 1 bis 5, worin die Zusammensetzung den als einen freien Liganden oder einen Übergangsmetall-substituierbaren Metallligandenkomplex vorliegenden Liganden und eine Quelle für Übergangsmetall umfasst.

10. Bleichzusammensetzung nach einem vorangehenden Anspruch, worin Z1, Z2 und Z4 unabhängig einen gegebenenfalls substituierten heterocyclischen Ring oder einen gegebenenfalls substituierten heteroaromatischen Ring, ausgewählt aus Pyridin, Pyrimidin, Pyrazin, Pyrazol, Imidazol, Benzimidazol, Chinolin, Chinoxalin, Triazol, Isochinolin, Carbazol, Indol, Isoindol, Oxazol und Thiazol, wiedergibt.

11. Bleichzusammensetzung nach einem vorangehenden Anspruch, worin Z1, Z2 und Z4 unabhängig Gruppen wiedergeben, ausgewählt aus gegebenenfalls substituiertem Pyridin-2-yl, gegebenenfalls substituiertem Imidazol-2-yl, gegebenenfalls substituiertem Imidazol-4-yl, gegebenenfalls substituiertem Pyrazol-1-yl und gegebenenfalls substituiertem Chinolin-2-yl.

12. Bleichzusammensetzung nach einem vorangehenden Anspruch, worin Z1, Z2 und Z4 jeweils unabhängig gegebenenfalls substituiertes Pyridin-2-yl wiedergeben.

13. Bleichzusammensetzung nach einem vorangehenden Anspruch, worin Z1, Z2 und Z4 gegebenenfalls mit einer Gruppe, ausgewählt aus C₁₋₄-Alkyl, Aryl, Arylalkyl, Heteroaryl, Methoxy, Hydroxy, Nitro, Amino, Carboxyl, Halogen und Carbonyl, substituiert sind.

14. Bleichzusammensetzung nach einem vorangehenden Anspruch, worin einer oder mehrere von Z1, Z2 und Z4 mit einer Methylgruppe substituiert sind.

15. Bleichzusammensetzung nach einem vorangehenden Anspruch, worin die Gruppen Z1 identische Gruppen wiedergeben.

16. Bleichzusammensetzung nach einem vorangehenden Anspruch, worin R5, R6, R7, R8 unabhängig eine Gruppe, ausgewählt aus -H, Hydroxy-C₀-C₂₀-alkyl, Halogen-C₀-C₂₀-alkyl, Nitroso, Formyl-C₀-C₂₀-alkyl, Carboxyl-C₀-C₂₀-alkyl, und Estern und Salzen davon, Carbamoyl-C₀-C₂₀-alkyl, Sulfo-C₀-C₂₀-alkyl, und Estern und Salzen davon, Sulfamoyl-C₀-C₂₀-alkyl, Amino-C₀-C₂₀-alkyl, Aryl-C₀-C₂₀-alkyl, C₀-C₂₀-Alkyl, Alkoxy-C₀-C₈-alkyl, Carbonyl-C₀-C₆-alkoxy und C₀-C₂₀-Alkylamid, wiedergeben.

17. Bleichzusammensetzung nach einem vorangehenden Anspruch, worin jedes Q1 eine kovalente Bindung oder C₁-C₄-Alkylen, vorzugsweise eine kovalente Bindung, Methylen oder Ethylen, bevorzugter eine kovalente Bindung, wiedergibt.

18. Bleichzusammensetzung nach einem vorangehenden Anspruch, worin jedes Q3 eine kovalente Bindung oder C₁-C₄-Alkylen, vorzugsweise eine kovalente Bindung, wiedergibt.

19. Bleichzusammensetzung nach einem vorangehenden Anspruch, worin T Wasserstoff, Hydroxy, Methyl, Ethyl, Benzyl oder Methoxy wiedergibt.

20. Bleichzusammensetzung nach einem der Ansprüche 1 bis 19, worin U eine koordinierende Gruppe der allgemeinen Formel (II) wiedergibt:

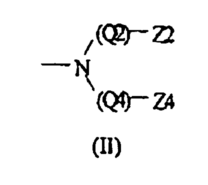

21. Bleichzusammensetzung nach Anspruch 20, worin Z2 einen gegebenenfalls substituierten heterocyclischen Ring oder einen gegebenenfalls substituierten heteroaromatischen Ring, ausgewählt aus Pyridin, Pyrimidin, Pyrazin, Pyrazol, Imidazol, Benzimidazol, Chinolin, Chinoxalin, Triazol, Isochinolin, Carbazol, Indol, Isoindol, Oxazol und Thiazol, vorzugsweise gegebenenfalls substituiertem Pyridin-2-yl oder gegebenenfalls substituiertem Benzimidazol-2-yl, wiedergibt, und worin Z4 einen gegebenenfalls substituierten heterocyclischen Ring oder einen gegebenenfalls substituierten heteroaromatischen Ring, ausgewählt aus Pyridin, Pyrimidin, Pyrazin, Pyrazol, Imidazol, Benzimidazol, Chinolin, Chinoxalin, Triazol, Isochinolin, Carbazol, Indol, Isoindol, Oxazol und Thiazol, vorzugsweise gegebenenfalls substituiertes Pyridin-2-yl oder eine nicht-koordinierende Gruppe, ausgewählt aus Wasserstoff, Hydroxy, Alkoxy, Alkyl, Alkenyl, Cycloalkyl, Aryl oder Benzyl, wiedergibt.

22. Bleichzusammensetzung nach Anspruch 21, worin L einen Liganden wiedergibt, ausgewählt aus:
1,1-Bis(pyridin-2-yl)-N-methyl-N-(pyridin-2-ylmethyl)methylamin;
1,1-Bis(pyridin-2-yl)-N,N-bis(6-methyl-pyridin-2-ylmethyl)methylamin;
1,1-Bis(pyridin-2-yl)-N,N-bis(5-carboxymethyl-pyridin-2-ylmethyl)methylamin;
1,1-Bis(pyridin-2-yl)-1-benzyl-N,N-bis(pyridin-2-ylmethyl)methylamin und
1,1-Bis(pyridin-2yl)-N,N-bis(benzimidazol-2-ylmethyl)methylamin.

23. Bleichzusammensetzung nach Anspruch 20, worin Z4 eine Gruppe der allgemeinen Formel (IIa) 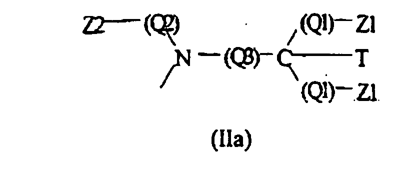 wiedergibt
und Q4 gegebenenfalls substituiertes Alkylen, vorzugsweise -CH₂-CHOH-CH₂- oder -CH₂-CH₂-CH₂-, wiedergibt.

24. Bleichzusammensetzung nach Anspruch 23, worin L den Liganden: 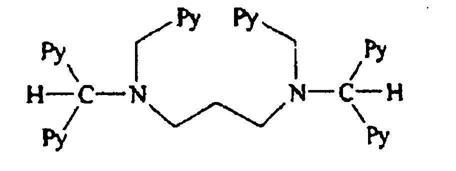 worin -Py Pyridin-2-yl wiedergibt, wiedergibt.

25. Bleichzusammensetzung nach einem der Ansprüche 1 bis 19, worin U eine koordinierende Gruppe der allgemeinen Formel (III): 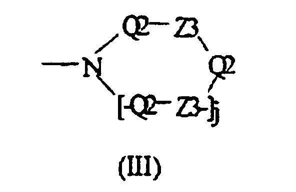 wiedergibt,
worin j 1 oder 2, vorzugsweise 1, ist.

26. Bleichzusammensetzung nach Anspruch 25, worin jedes Q2 -(CH₂)ₙ- (n=2-4) wiedergibt und jedes Z3 -N(R)- wiedergibt, worin R = -H oder C₁₋₄-Alkyl.

27. Bleichzusammensetzung nach Anspruch 26, worin L einen Liganden wiedergibt, ausgewählt aus: 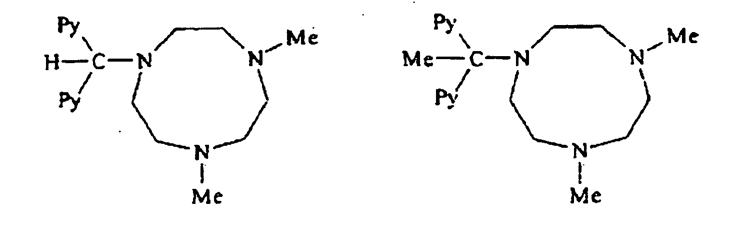 worin -Py Pyridin-2-yl wiedergibt.

28. Bleichzusammensetzung nach einem der Ansprüche 1 bis 19, worin U eine koordinierende Gruppe der allgemeinen Formel (IV): 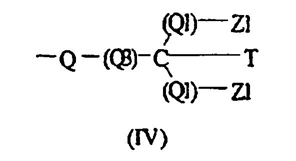 wiedergibt.

29. Bleichzusammensetzung nach Anspruch 28, worin Q -N(T)- (worin T= -H, Methyl oder Benzyl) oder Pyridindiyl wiedergibt.

30. Bleichzusammensetzung nach Anspruch 29, worin L einen Liganden wiedergibt, ausgewählt aus: 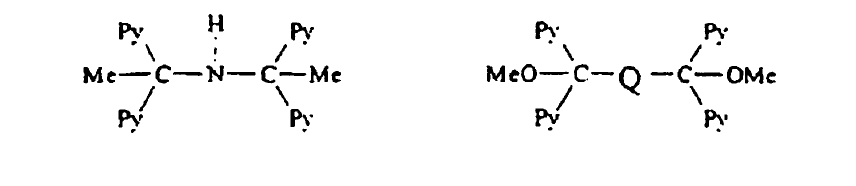 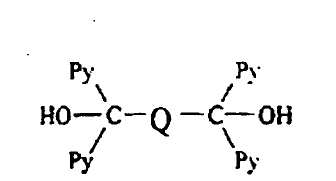 worin -Py Pyridin-2-yl wiedergibt und -Q- Pyridin-2,6-diyl wiedergibt.

31. Bleichzusammensetzung nach einem vorangehenden Anspruch, worin die Zusammensetzung ein Gemisch des Liganden L und eines Metallsalzes MXₙ, worin n=1-5, vorzugsweise 1-3, umfasst.

32. Ligand L wie in einem der vorangehenden Ansprüche definiert, mit der Maßgabe, dass U eine nicht-koordinierte Gruppe T oder eine koordinierte Gruppe der Formel (II) oder (III) wiedergibt, und, wenn U eine koordinierende Gruppe der Formel (II) wiedergibt und Z1=Z2=Z4=unsubstituiertes Pyridin-2-yl, T nicht Wasserstoff, Methyl oder Benzyl darstellt.

33. Ligand, ausgewählt aus:
1,1-Bis(pyridin-2-yl)-N-methyl-N(pyridin-2-ylmethyl)methylamin;
1,1-Bis(pyridin-2-yl)-N,N-bis(6-methyl-pyridin-2-ylmethyl)methylamin;
1,1-Bis(pyridin-2-yl)-N,N-bis(5-carboxymethyl-pyridin-2-yl)methylamin;
1,1-Bis(pyridin-2yl)-N,N-bis(benzimidazol-2-ylmethyl)methylamin;
2,6-Bis(pyridin-2-ylmethyl)-1,1,7,7-tetrakis(pyridin-2-yl)-2,6-diazaheptan, oder einen Ligand, ausgewählt aus: 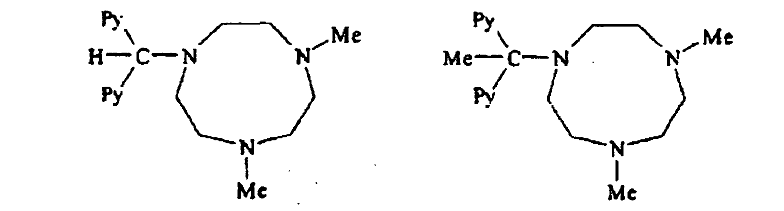 worin -Py Pyridin-2-yl wiedergibt.

34. Übergangsmetallkomplex der allgemeinen Formel (A1):
[MₐLₖXₙ]Yₘ (A1)
worin:
M ein Metall, ausgewählt aus Mn(II)-(III)-(IV)-(V), Cu(I)-(II)-(III), Fe(II)-(III)-(IV)-(V), Co(I)-(II)-(III), Ti(II)-(III)-(IV), V(II)-(III)-(IV)-(V), Mo(II)-(III)-(IV)-(V)-(VI) und W(IV)-(V)-(VI), wiedergibt;
X eine koordinierende Spezies, ausgewählt aus beliebigen ein-, zwei- oder dreifach geladenen Anionen und beliebigen neutralen Molekülen, die das Metall in einer ein-, zwei- oder dreizähnigen Weise koordinieren können, wiedergibt;
Y beliebiges nicht-koordiniertes Gegenion wiedergibt;
a eine ganze Zahl von 1 bis 10 wiedergibt;
k eine ganze Zahl von 1 bis 10 wiedergibt;
n eine ganze Zahl von 1 bis 10 wiedergibt;
m null oder eine ganze Zahl von 1 bis 20 wiedergibt und
L einen Liganden, wie in Anspruch 32 oder 33 definiert, oder sein protoniertes oder deprotoniertes Analoges wiedergibt.

35. Verfahren zum Bleichen eines Substrats, umfassend Auftragen eines Liganden, der einen Komplex mit einem Übergangsmetall bildet, auf das Substrat in einem wässrigen Medium, wobei der Komplex das Bleichen des Substrats durch atmosphärischen Sauerstoff katalysiert, worin der Ligand wie in einem der Ansprüche 1 bis 33 definiert ist.

36. Verfahren nach Anspruch 35, wobei die Mehrheit der bleichenden Spezies in dem Medium (auf einer Äquivalentgewichtsbasis) von dem atmosphärischen Sauerstoff abgeleitet ist.

37. Verfahren nach Anspruch 35 oder Anspruch 36, wobei das Medium im Wesentlichen frei von Persauerstoffbleichmittel oder einem auf Peroxy basierenden oder erzeugenden Bleichmittelsystem ist.

38. Verfahren nach einem der Ansprüche 35 bis 37, wobei das wässrige Medium gerührt wird.

39. Verfahren nach einem der Ansprüche 35 bis 38, wobei das Medium wie in einem der Ansprüche 2 bis 5 definiert ist.

40. Verwendung eines Liganden, der mit einem Übergangsmetall einen Komplex bildet, als ein katalytisches Bleichmittel für ein Substrat in einem wässrigen Medium, das im Wesentlichen frei von Persauerstoffbleichmittel oder einem auf Persauerstoff basierenden oder erzeugenden Bleichmittelsystem ist, wobei der Komplex Bleichen des Substrats durch atmosphärischen Sauerstoff katalysiert, wobei der Ligand wie in einem der Ansprüche 1 bis 33 definiert ist.

41. Trockenes Textil, auf das ein Ligand, wie in einem der Ansprüche 1 bis 33 definiert, darauf aufgetragen oder abgeschieden ist, wodurch das Bleichen durch atmosphärischen Sauerstoff auf dem Textil katalysiert wird.

## Revendications

1. Composition de blanchiment comprenant, dans un milieu aqueux, de l'oxygène atmosphérique et un ligand qui forme un complexe avec un métal de transition, le complexe catalysant le blanchiment d'un substrat par l'oxygène atmosphérique, dans laquelle le milieu aqueux est substantiellement dénué de système de blanchiment peroxygéné ou de système de blanchiment générant ou à base de peroxy,
dans laquelle le ligand forme un complexe de formule générale (A1) :
[MₐLₖXₙ]Yₘ (A1)
dans laquelle :
M représente un métal choisi parmi Mn(II)-(III)-(IV)-(V), Cu(I)-(II)-(III), Fe(II)-(III)-(IV)-(V), Co(I)-(II)-(III), Ti(II)-(III)-(IV), V(II)-(III)-(IV)-(V), Mo(II)-(III)-(IV)-(V)-(VI) et W(IV)-(V)-(VI) ;
X représente une espèce coordinante choisie parmi des anions mono-, bi- ou tri-chargés quelconques et des molécules neutres quelconques capables de coordiner le métal d'une manière mono-, bi- ou tri-dentale ;
Y représente un contre-ion quelconque non coordiné ;
a représente un entier de 1 à 10 ;
k représente un entier de 1 à 10 ;
n représente un entier de 1 à 10 ;
m représente zéro ou un entier de 1 à 20 ; et
L représente un ligand de formule générale (I),
ou son analogue protoné ou déprotoné : 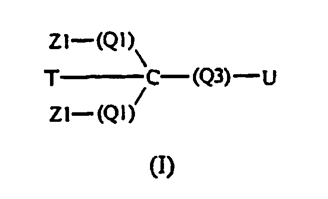 dans lequel
les groupes Z1 représentent indépendamment un groupe coordinant choisi parmi un hydroxy, amino, -NHR ou -N(R)₂ (dans lequel R = alkyle en C₁ à C₆), carboxylate, amido, -NH-C(NH)NH₂, hydroxyphényle, un cycle hétérocyclique éventuellement substitué par un ou plusieurs groupes fonctionnels E ou un cycle hétéroaromatique éventuellement substitué par un ou plusieurs groupes fonctionnels E, le cycle hétéroaromatique étant choisi parmi les groupes pyridine, pyrimidine, pyrazine, pyrazole, imidazole, benzimidazole, quinoléine, quinoxaline, triazole, isoquinoléine, carbazole, indole, isoindole, oxazole et thiazole ;
Q1 et Q3 représentent indépendamment un groupe de formule : 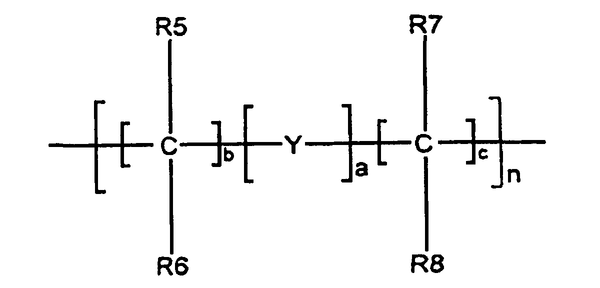 dans laquelle
5 ≥ a + b + c ≥ 1 ; a = 0-5 ; b = 0-5 ; c = 0-5 ; n = 0 ou 1 (de préférence n = 0) ;
Y représente indépendamment un groupe choisi parmi -O-, -S-, -SO-, -SO₂-, -C(O)-, arylène, alkylène, hétéroarylène, hétérocycloalkylène, -(G)P-, -P(O)- et -(G)N-, dans lequel G est choisi parmi un hydrogène, alkyle, aryle, arylalkyle, cycloalkyle, chacun sauf l'hydrogène étant éventuellement substitué par un ou plusieurs groupes fonctionnels E ;
R5, R6, R7, R8 représentent indépendamment un groupe choisi parmi un hydrogène, hydroxyle, halogène, -R et -OR, dans lesquels R représente un alkyle, alcényle, cycloalkyle, hétérocycloalkyle, aryle, hétéroaryle ou un groupe dérivé de carbonyle, R étant éventuellement substitué par un ou plusieurs groupes fonctionnels E,
ou R5 ensemble avec R6, ou R7 ensemble avec R8, ou les deux, représentent l'oxygène,
ou R5 ensemble avec R7 et/ou indépendamment R6 ensemble avec R8, ou R5 ensemble avec R8 et/ou indépendamment R6 ensemble avec R7, représentent un alkylène en C₁ à C₆ éventuellement substitué par un alkyle en C₁ à C₄, -F, -Cl, -Br ou -I ;
E représente indépendamment un groupe fonctionnel choisi parmi -F, -Cl, -Br, -I, -OH, -OR', -NH₂, -NHR', -N(R')₂, -N(R')₃, -C(O)R', -OC(O)R', -COOH, -COO⁻(Na⁺,K⁺) , -COOR' , -C(O)NH₂, -C (O) NHR', -C(O)N(R')₂, hétéroaryle, -R', -SR', -SH, -P(R')₂, -P(O) (R')₂, -P(O) (OH)₂, P(O) (OR')₂, -NO₂, -SO₃H, -SO₃⁻(Na⁺,K⁺), -S(O)₂R', -NHC(O)R' et -N(R')C(O)R', dans lesquels R' représente un cycloalkyle, aryle, arylalkyle ou alkyle éventuellement substitué par -F, -C1, -Br, -I, -NH₃⁺, -SO₃H, -SO₃⁻(Na⁺,K⁺), -COOH, -COO⁻(Na⁺,K⁺), -P(O) (OH)₂ ou P (O) (O⁻(Na⁺,K⁺))₂ ;
T représente un groupe non coordiné choisi parmi un hydrogène, hydroxyle, halogène, -R et -OR, dans lesquels R représente un alkyle, alcényle, cycloalkyle, hétérocycloalkyle, aryle, hétéroaryle ou un groupe dérivé de carbonyle, R étant éventuellement substitué par un ou plusieurs groupes fonctionnels E ;
U représente un groupe non coordiné T indépendamment défini comme ci-dessus ou un groupe coordinant de formule générale (II), (III) ou (IV) : 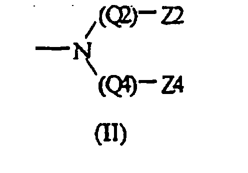 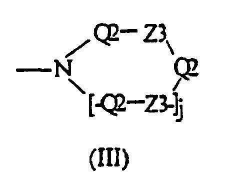 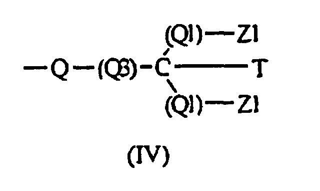 dans laquelle
Q2 et Q4 sont indépendamment définis comme pour Q1 et Q3 ;
Q représente -N(T)- (dans lequel T est indépendamment défini comme ci-dessus), ou un cycle hétérocyclique éventuellement substitué ou un cycle hétéroaromatique éventuellement substitué choisi parmi les groupes pyridine, pyrimidine, pyrazine, pyrazole, imidazole, benzimidazole, quinoléine, quinoxaline, triazole, isoquinoléine, carbazole, indole, isoindole, oxazole et thiazole ;
Z2 est indépendamment défini comme pour Z1 ;
les groupes Z3 indépendamment représentent -N(T)- (dans lequel T est indépendamment défini comme ci-dessus) ;
Z4 représente un groupe coordinant ou non coordinant choisi parmi un hydrogène, hydroxyle, halogène, -NH-C(NH)NH₂, -R et -OR, dans lesquels R = alkyle, alcényle, cycloalkyle, hétérocycloalkyle, aryle, hétéroaryle ou un groupe dérivé de carbonyle, R étant éventuellement substitué par un ou plusieurs groupes fonctionnels E, ou Z4 représente un groupe de formule générale (IIa) : 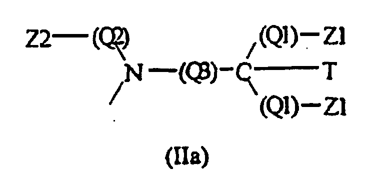 et
1 ≤ j < 4.

2. Composition de blanchiment selon la revendication 1, dans laquelle le milieu a une valeur de pH dans la gamme de pH 6 à 11, de préférence dans la gamme de pH 8 à 10.

3. Composition de blanchiment selon la revendication 1 ou la revendication 2, dans laquelle le milieu est essentiellement dénué d'un séquestrant des métaux de transition.

4. Composition de blanchiment selon l'une quelconque des revendications 1 à 3, dans laquelle le milieu comprend en outre un agent tensio-actif.

5. Composition de blanchiment selon l'une quelconque des revendications 1 à 4, dans laquelle le milieu comprend en outre un adjuvant.

6. Composition de blanchiment selon l'une quelconque des revendications 1 à 5, dans laquelle la composition comprend un complexe préformé du ligand et d'un métal de transition.

7. Composition de blanchiment selon l'une quelconque des revendications 1 à 5, dans laquelle le ligand est présent sous forme de ligand libre qui se complexe avec un métal de transition présent dans l'eau.

8. Composition de blanchiment selon l'une quelconque des revendications 1 à 5, dans laquelle le ligand est présent sous forme de ligand libre qui se complexe avec un métal de transition présent dans le substrat.

9. Composition de blanchiment selon l'une quelconque des revendications 1 à 5, dans laquelle la composition comprend le ligand présent sous forme d'un ligand libre ou d'un complexe métal de transition-métal substituable-ligand, et une source de métal de transition.

10. Composition de blanchiment selon l'une quelconque des revendications précédentes, dans laquelle Z1, Z2 et Z4 représentent indépendamment un cycle hétérocyclique éventuellement substitué ou un cycle hétéroaromatique éventuellement substitué choisi parmi les groupes pyridine, pyrimidine, pyrazine, pyrazole, imidazole, benzimidazole, quinoléine, quinoxaline, triazole, isoquinoléine, carbazole, indole, isoindole, oxazole et thiazole.

11. Composition de blanchiment selon l'une quelconque des revendications précédentes, dans laquelle Z1, Z2 et Z4 représentent indépendamment des groupes choisis parmi le pyridin-2-yle éventuellement substitué, imidazol-2-yle éventuellement substitué, imidazol-4-yle éventuellement substitué, pyrazol-1-yle éventuellement substitué et quinoléin-2-yle éventuellement substitué.

12. Composition de blanchiment selon l'une quelconque des revendications précédentes, dans laquelle Z1, Z2 et Z4 représentent chacun le pyridin-2-yle éventuellement substitué.

13. Composition de blanchiment selon l'une quelconque des revendications précédentes, dans laquelle Z1, Z2 et Z4 sont éventuellement substitués par un groupe choisi parmi un alkyle en C₁ à C₄, aryle, arylalkyle, hétéroaryle, méthoxy, hydroxy, nitro, amino, carboxyle, halogéno et carbonyle.

14. Composition de blanchiment selon l'une quelconque des revendications précédentes, dans laquelle un ou plusieurs de Z1, Z2 et Z4 sont substitués par un groupe méthyle.

15. Composition de blanchiment selon l'une quelconque des revendications précédentes, dans laquelle les groupes Z1 représentent des groupes identiques.

16. Composition de blanchiment selon l'une quelconque des revendications précédentes, dans laquelle R5, R6, R7, R8 représentent indépendamment un groupe choisi parmi -H, un hydroxy-alkyle en C₀ à C₂₀, halogéno-alkyle en C₀ à C₂₀, nitroso, formyl-alkyle en C₀ à C₂₀, carboxyl-alkyle en C₀ à C₂₀ et leurs esters et sels, carbamoyl-alkyle en C₀ à C₂₀, sulfo-alkyle en C₀ à C₂₀ et leurs esters et sels, sulfamoyl-alkyle en C₀ à C₂₀, amino-alkyle en C₀ à C₂₀, aryl-alkyle en C₀ à C₂₀, alkyle en C₀ à C₂₀, alcoxy-alkyle en C₀ à C₈, carbonyl-alcoxy en C₀ à C₆ et alkylamide en C₀ à C₂₀.

17. Composition de blanchiment selon l'une quelconque des revendications précédentes, dans laquelle chaque Q1 représente une liaison covalente ou un alkylène en C₁ à C₄, de préférence une liaison covalente, un méthylène ou éthylène, mieux encore une liaison covalente.

18. Composition de blanchiment selon l'une quelconque des revendications précédentes, dans laquelle chaque Q3 représente une liaison covalente ou un alkylène en C₁ à C₄, de préférence une liaison covalente.

19. Composition de blanchiment selon l'une quelconque des revendications précédentes, dans laquelle T représente un hydrogène, hydroxy, méthyle, éthyle, benzyle ou méthoxy.

20. Composition de blanchiment selon l'une quelconque des revendications 1 à 19, dans laquelle U représente un groupe coordinant de formule générale (II) :

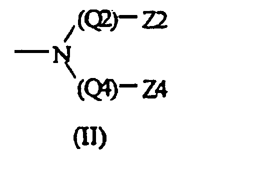

21. Composition de blanchiment selon la revendication 20, dans laquelle Z2 représente un cycle hétérocyclique éventuellement substitué ou un cycle hétéroaromatique éventuellement substitué choisi parmi les groupes pyridine, pyrimidine, pyrazine, pyrazole, imidazole, benzimidazole, quinoléine, quinoxaline, triazole, isoquinoléine, carbazole, indole, isoindole, oxazole et thiazole, de préférence pyridin-2-yle éventuellement substitué ou benzimidazol-2-yle éventuellement substitué, et dans laquelle Z4 représente un cycle hétérocyclique éventuellement substitué ou un cycle hétéroaromatique éventuellement substitué choisi parmi les groupes pyridine, pyrimidine, pyrazine, pyrazole, imidazole, benzimidazole, quinoléine, quinoxaline, triazole, isoquinoléine, carbazole, indole, isoindole, oxazole et thiazole, de préférence pyridin-2-yle éventuellement substitué, ou un groupe non coordinant choisi parmi un hydrogène, hydroxy, alcoxy, alkyle, alcényle, cycloalkyle, aryle ou benzyle.

22. Composition de blanchiment selon la revendication 21, dans laquelle L représente un ligand choisi parmi :
1,1-bis(pyridin-2-yl)-N-méthyl-N-(pyridin-2-ylméthyl)-méthylamine ;
1,1-bis(pyridin-2-yl)-N,N-bis(6-méthyl-pyridin-2-yl-méthyl)méthylamine ;
1,1-bis(pyridin-2-yl)-N,N-bis(5-carboxyméthyl-pyridin-2-ylméthyl)méthylamine ;
1,1-bis(pyridin-2-yl)-1-benzyl-N,N-bis(pyridin-2-yl-méthyl)méthylamine ; et
1,1-bis(pyridin-2-yl)-N,N-bis(benzimidazol-2-yl-méthyl)-méthylamine.

23. Composition de blanchiment selon la revendication 20, dans laquelle Z4 représente un groupe de formule générale (IIa) : 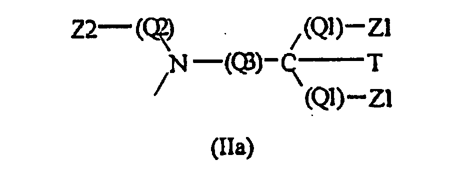 et Q4 représente un alkylène éventuellement substitué, de préférence -CH₂-CHOH-CH₂- ou -CH₂-CH₂-CH₂-.

24. Composition de blanchiment selon la revendication 23, dans laquelle L représente le ligand : 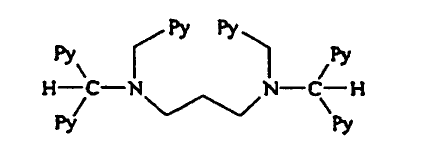 dans lequel -Py représente le pyridin-2-yle.

25. Composition de blanchiment selon l'une quelconque des revendications 1 à 19, dans laquelle U représente un groupe coordinant de formule générale (III) : 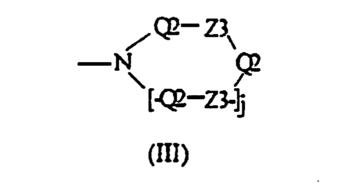 dans laquelle j est 1 ou 2, de préférence 1.

26. Composition de blanchiment selon la revendication 25, dans laquelle chaque Q2 représente -(CH₂)ₙ- (n = 2-4), et chaque Z3 représente -N(R)- dans lequel R = -H ou alkyle en C₁ à C₄.

27. Composition de blanchiment selon la revendication 26, dans laquelle L représente un ligand choisi parmi : 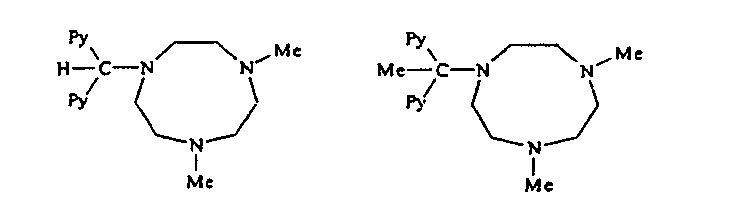 dans lesquels -Py représente le pyridin-2-yle.

28. Composition de blanchiment selon l'une quelconque des revendications 1 à 19, dans laquelle U représente un groupe coordinant de formule générale (IV) :

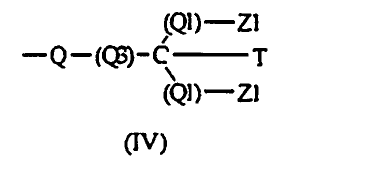

29. Composition de blanchiment selon la revendication 28, dans laquelle chaque Q représente -N(T)- (dans lequel T = -H, méthyle ou benzyle) ou un pyridine-diyle.

30. Composition de blanchiment selon la revendication 29, dans laquelle L représente un ligand choisi parmi : 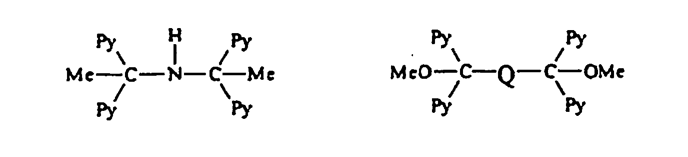 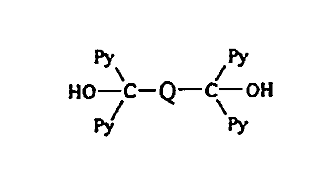 dans lesquels -Py représente le pyridin-2-yle, et Q représente le pyridin-2,6-diyle.

31. Composition de blanchiment selon l'une quelconque' des revendications précédentes, dans laquelle la composition comprend un mélange du ligand L et d'un sel métallique MXₙ dans lequel n = 1-5, de préférence 1-3.

32. Un ligand L tel que défini dans l'une quelconque des revendications précédentes sous réserve que U représente un groupe non coordiné T ou un groupe coordiné de formule (II) ou (III), et si U représente un groupe coordinant de formule (II) et Z1 = Z2 = Z4 = pyridin-2-yle non substitué, T n'est pas un hydrogène, méthyle ni benzyle.

33. Ligand choisi parmi :
1,1-bis(pyridin-2-yl)-N-méthyl-N-(pyridin-2-ylméthyl)-méthylamine ;
1,1-bis(pyridin-2-yl)-N,N-bis(6-méthyl-pyridin-2-yl-méthyl)méthylamine ;
1,1-bis(pyridin-2-yl)-N,N-bis(5-carboxyméthyl-pyridin-2-ylméthyl)méthylamine ;
1,1-bis(pyridin-2-yl)-N,N-bis(benzimidazol-2-yl-méthyl)méthylamine ;
2,6-bis(pyridin-2-ylméthyl)-1,1,7,7-tétrakis(pyridin-2-yl)-2,6-diazaheptane ; ou un ligand choisi parmi : 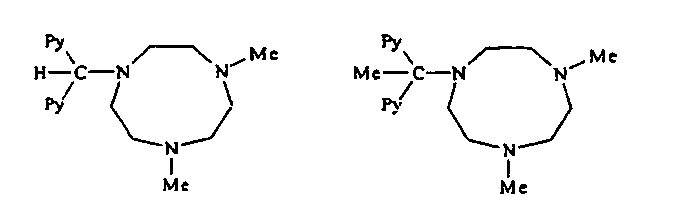 dans lesquels -Py représente le pyridin-2-yle.

34. Complexe de métal de transition de formule générale (A1) :
[MₐLₖXₙ]Yₘ (A1)
dans laquelle :
M représente un métal choisi parmi Mn(II)-(III)-(IV)-(V), Cu(I)-(II)-(III), Fe(II)-(III)-(IV)-(V), Co(I)-(II)-(III), Ti(II)-(III)-(IV), V(II)-(III)-(IV)-(V), Mo(II)-(III)-(IV)-(V)-(VI) et W(IV)-(V)-(VI) ;
X représente une espèce coordinante choisie parmi des anions quelconques mono-, bi- ou tri-chargés et des molécules neutres quelconques capables de coordiner le métal d'une manière mono-, bi- ou tri-dentale ;
Y représente un contre-ion quelconque non coordiné ;
a représente un entier de 1 à 10 ;
k représente un entier de 1 à 10 ;
n représente un entier de 1 à 10 ;
m représente zéro ou un entier de 1 à 20 ; et
L représente un ligand tel que défini à la revendication 32 ou 33 ou son analogue protoné ou déprotoné.

35. Procédé de blanchiment d'un substrat comprenant l'application au substrat, dans un milieu aqueux, d'un ligand qui forme un complexe avec un métal de transition, le complexe catalysant le blanchiment du substrat par l'oxygène atmosphérique, le ligand étant tel que défini dans l'une quelconque des revendications 1 à 33.

36. Procédé selon la revendication 35, dans lequel la majorité des espèces de blanchiment dans le milieu (ou un équivalent sur la base du poids) dérive de l'oxygène atmosphérique.

37. Procédé selon la revendication 35 ou la revendication 36, dans lequel le milieu est essentiellement dénué d'agent de blanchiment peroxygène ou de système de blanchiment générant ou à base de peroxy.

38. Procédé selon l'une quelconque des revendications 35 à 37, dans lequel le milieu aqueux est agité.

39. Procédé selon l'une quelconque des revendications 35 à 38, dans lequel le milieu est tel que défini dans l'une quelconque des revendications 2 à 5.

40. Utilisation d'un ligand qui forme un complexe avec un métal de transition comme agent de blanchiment catalytique pour un substrat dans un milieu aqueux essentiellement dénué d'agent de blanchiment peroxygène ou de système de blanchiment générant ou à base de peroxy, le complexe catalysant le blanchiment du substrat par l'oxygène atmosphérique, le ligand étant tel que défini dans l'une quelconque des revendications 1 à 33.

41. Textile sec ayant un ligand tel que défini dans l'une quelconque des revendications 1 à 33 appliqué ou déposé dessus, ce faisant le blanchiment par l'oxygène atmosphérique est catalysé sur le textile.
